# EUROPEAN PATENT APPLICATION

(11) **EP 4 066 854 A1**
(43) Date of publication of application: **05.10.2022**
(21) Application number: 21165674.9
(22) Date of filing: 29.03.2021
(51) Int. Cl.: A61K 39/09, C07K 14/315, A61P 31/04

(54) **IMMUNOGENIC FUSION PROTEIN**

(71) Applicant: MinervaX, 2200 Copenhagen N (DK)
(72) Inventor: PEDERSEN FISCHER, Per Bo, 2000 FREDERIKSBERG (DK)
(74) Representative: Brann AB

(57) **Abstract**

The present invention relates to an immunogenic fusion protein capable of eliciting protective immunity against group B Streptococcus, the immunogenic fusion protein comprising or consisting of an amino acid sequence consisting of: i. a first amino acid sequence part consisting of 170 to 178 amino acids, preferably 174 to 175 amino acids, and having at least 90% sequence identity with the amino acid sequence shown in SEQ ID NO 7; ii. a second amino acid sequence part consisting of 165 to 174 amino acids, preferably 169 to 170 amino acids, and having at least 90% sequence identity with the amino acid sequence shown in SEQ ID NO 14; and optionally: iii. a linker amino acid sequence part consisting of 1 to 20 amino acids and separating the first amino acid sequence part from the second amino acid part. The immunogenic fusion protein preferably consists of 335 to 372 amino acids, preferably 343 to 353 amino acids, more preferably 343 to 347 amino acids. The invention further pertains to nucleic acid molecule encoding the immunogenic fusion protein; a vector; a host cell; a vaccine; and a method of vaccination against group B Streptococcus infection or treating a group B Streptococcus infection.

## Description

### FIELD OF INVENTION

The present invention relates to the fields of microbiology and vaccine technology and concerns the development of an immunogenic fusion protein capable of conferring immunity to group B Streptococcus infections. More particularly, the present invention relates to an immunogenic fusion protein which confers immunity to invasive strains of the group B Streptococcus. It further pertains to a nucleic acid molecule encoding the immunogenic fusion protein; a vector; a host cell; a vaccine; and a method of vaccination against group B Streptococcus infection or treating a group B Streptococcus infection.

### BACKGROUND OF THE INVENTION

Group B Streptococcus (*Streptococcus agalactiae)* (GBS) is the major cause of invasive bacterial infections, including meningitis, in the neonatal period. In the United States alone, there are now about 5000 cases per year of invasive disease caused by this bacterium. These infections have an overall mortality of about 10%, and many of the infants that survive have permanent neurological sequelae. In view of this, a large effort has been made to find methods of prevention and treatment and to analyze the mechanisms by which GBS cause infections.

GBS can also cause mastitis in cows, a bovine disease that is of considerable economical importance. Development of a vaccine against GBS infections is therefore of interest also in veterinary medicine.

About 20 % of all women are vaginal carriers of GBS, and vertical transmission from the maternal genital tract is probably the most common source of infection in neonatal disease caused by this bacterium. However, only about 1 % of the infants that are colonized by the GBS at birth are afflicted by serious infection. Other factors than exposure to the bacterium during birth must therefore contribute to the development of neonatal disease.

Group B streptococcal strains are divided into nine serotypes (la, Ib, and II-VIII) based on the structure of the polysaccharide capsule. The four "classical" serotypes la, Ib, II, and III occur in roughly equal proportions among strains in the normal flora, but type III is the clinically most important serotype, in particular because it causes most cases of meningitis. Because the capsule is a known virulence factor, it has been studied in considerable detail, in particular in type III strains. Efforts have been made to develop a vaccine, in which the type III polysaccharide capsule would be an essential component.

EP 0 866 133 discloses a vaccine capable of protecting a recipient from infection caused by group B *Streptococcus.* The invention is directed to the use of a combination of a polysaccharide and a fragment of the epsilon protein. It further discloses that epidemiological data suggest that the type-specific capsule plays an important role in the immunity to group B *Streptococcus* infections (see page 7 line 2-3). Additionally, there are a number of different combinations between different proteins and the polysaccharide mentioned within the application, but all the claims comprise a polysaccharide which shows the importance of that particular component. However, use of the polysaccharide capsule as a vaccine may give problems due to cross reactions with human tissues (Pritchard et al., Infect Immun 1992. 60: 1598). It would therefore be very valuable if one could develop a vaccine based on proteins rather than on polysaccharides.

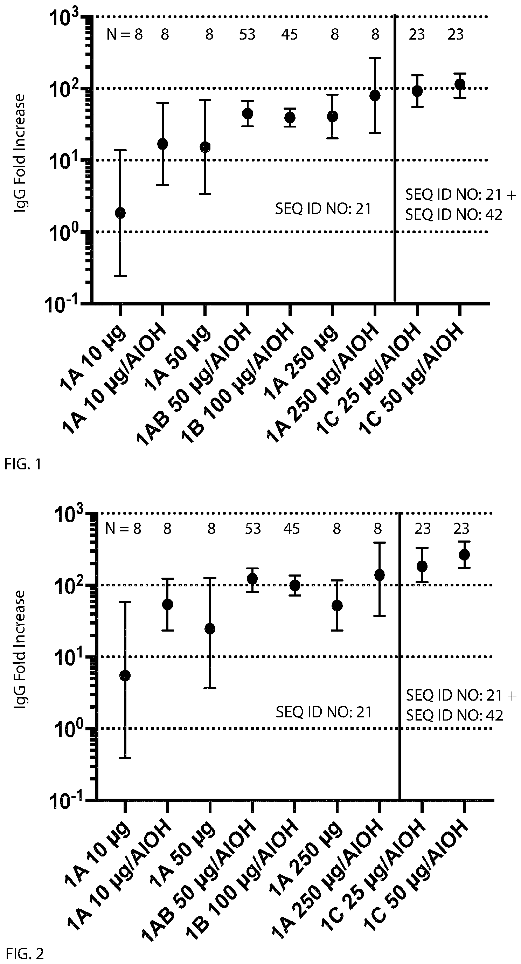

The document Gravekamp et al., Infection and Immunity, Dec 1997, p 5216-5221 discloses the evaluation of the immunogenicity as well as protection of the number of repeats of the alpha (α) C protein as well as the N-terminal part alone. It was found that the immunogenicity decreased with increasing number of repeats (se Fig 2B). However, it was also found in a protection assay that the antibodies against the N-terminal region were predominantly responsible for the protection compared to antibodies against the N-terminal region (see page 5219 left column, line 6 from the bottom, and page 5220 right column lines 26-29).

WO 9410317 describes the use of the alpha protein, a GBS surface protein, in the development of a conjugate vaccine. A drawback with this protein is that it usually is not expressed by type III strains, which are the cause of many serious GBS infections. Hence, a protective immunity against these strains will not be evoked by an alpha protein vaccine.

WO 9421685 describes the use of the Rib protein, a GBS surface protein, in the development of a vaccine. This protein elicits immunity when administered with alum. However, the Rib protein has the disadvantage that it does not evoke a protective immunity against all GBS strains.

The document Lindahl et al, Nonimmunodominant Regions Are Effective As Building Blocks In A Streptococcal Fusion Protein Vaccine, Cell Host & Microbe 2, 427-434, December 2007, discloses a fusion protein comprising N-terminal regions of the group B Streptococcus surface proteins Rib and AlpC.

Similarly, WO 2008127179 describes a fusion protein comprising at least one first N-terminal region fragment of a group B Streptococcus surface protein or analogue, homologue, derivative or immunologically related amino acid sequence or fragments thereof, which is fused to at least one second N-terminal region fragment of a group B Streptococcus surface protein or analogue, homologue, derivative or immunologically related amino acid sequence or fragments thereof, wherein the first and second at least one N-terminal region fragments of group B Streptococcus surface proteins derive from different group B Streptococcus strains, and wherein the fusion protein is capable of eliciting protective immunity against group B Streptococcus.

Despite the advances in the progress towards a vaccine suitable for prevention of GBS disease, there is still a need for further methods and vaccines for prevention and treatment of GBS infections. Thus, there remains a need to explore vaccines strategies capable of eliciting protective immunity against a wide range of GBS stains.

Accordingly, it is a primary objective of the present invention to provide an immunogenic fusion protein which can be used in a vaccine capable of eliciting protective immunity against GBS infections.

It is a further objective of the present invention to provide a vaccine that elicits protective immunity against many clinically important GBS strains.

Another objective of the present invention is to provide a vaccine comprising a single, or a few, immunogenic fusion proteins that elicits protective immunity against GBS infections. A single or a few proteins has several advantages over a vaccine composed of numerous proteins, e.g., cost of production and safety.

The means of accomplishing each of the above objectives as well as others will become apparent from the description of the invention which follows hereafter.

### SUMMARY OF THE INVENTION

The present invention is based on further work, by the present inventor, on the fusion protein disclosed in WO 2008127179. Specifically, an immunogenic fusion protein with a shorter amino acid sequence of the N terminal regions of Rib and AlpC has been found to be suitable. Such an immunogenic fusion protein may be easier to produce and use for vaccination.

Additionally, codon optimized corresponding nucleic acid molecules encoding the immunogenic fusion protein have been devised. These may be provided in a vector. A host cell may in turn express the immunogenic fusion protein or comprise the vector. Each of the immunogenic fusion protein, the nucleic acid molecule, the vector, and the host cell may be used in a vaccine. The immunogenic fusion protein, the nucleic acid molecule, the vector, the host cell, and/or the vaccine may further be used in a method of vaccination against group B Streptococcus infection or treating a group B Streptococcus infection.

Thus, a first aspect of the present invention relates to an immunogenic fusion protein capable of eliciting protective immunity against group B Streptococcus, the immunogenic fusion protein comprising or consisting of an amino acid sequence consisting of:
i. a first amino acid sequence part consisting of 170 to 178 amino acids, preferably 174 to 175 amino acids, and having at least 90% sequence identity with the amino acid sequence shown in SEQ ID NO 7;
ii. a second amino acid sequence part consisting of 165 to 174 amino acids, preferably 169 to 170 amino acids, and having at least 90% sequence identity with the amino acid sequence shown in SEQ ID NO 14; and optionally:
iii. a linker amino acid sequence part consisting of 1 to 20 amino acids and separating the first amino acid sequence part from the second amino acid part,
and wherein the immunogenic fusion protein preferably consists of 335 to 372 amino acids, preferably 343 to 353 amino acids, more preferably 343 to 347 amino acids.

As noted above, the immunogenic fusion protein may be easier to produce and use for vaccination. The immunogenic fusion protein has the advantage that it is immunogenic even without adjuvant, however it can also be used with an adjuvant such as alum or Aluminum hydroxide (AlOH) for increased immunogenicity.

Another advantage with the immunogenic fusion protein is that it can be combined with a further immunogenic fusion protein comprising the N-terminal regions of Alp1 and Alp2. As seen in the examples, the combination of two such immunogenic fusion proteins provide a synergistic effect on the numbers of antibodies (antibody titers) that are obtained when the combination is used for immunization.

The present invention will be described in more detail below.

### BRIEF DESCRIPTION OF THE FIGURES

Fig. 1 shows IgG fold increase obtained 29 days after immunization using one dose of either an immunogenic fusion protein according to the first aspect of the present invention alone, or in combination with a further immunogenic fusion protein as encompassed by the vaccine according to the fifth aspect of the present invention, at different dosages with or without an adjuvant.
Fig. 2 shows IgG fold increase obtained 57 days from immunization using a first dose and a second dose of either an immunogenic fusion protein according to the first aspect of the present invention alone, or in combination with a further immunogenic fusion protein as encompassed by the vaccine according to the fifth aspect of the present invention, at different dosages with or without an adjuvant.

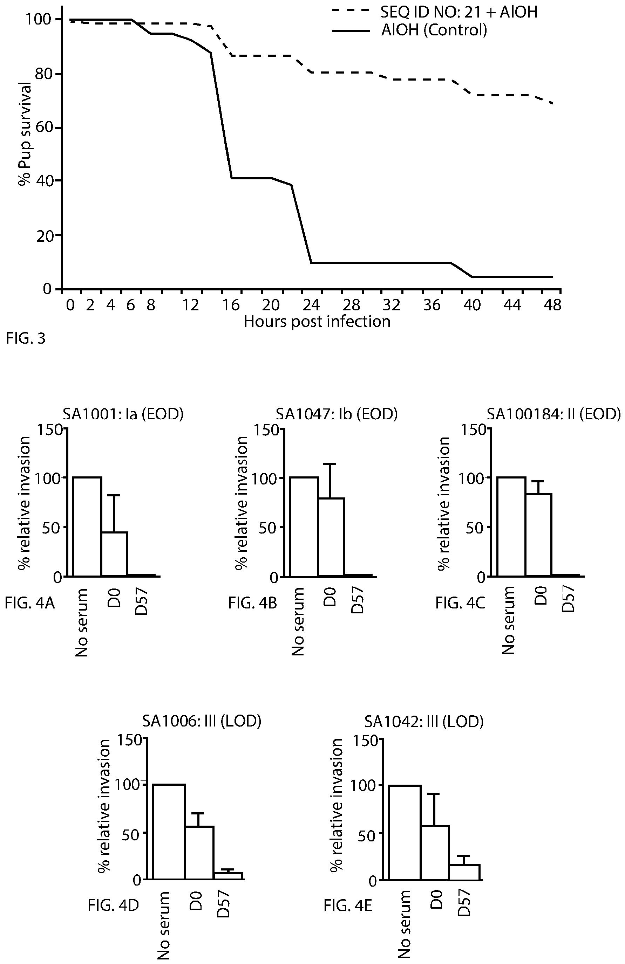

Fig. 3 shows survival of mice pups following neonatal bacterial infection with group B Streptococcus strain BM110 (8 x 10³ cfu). The pups were born from female mice having been immunized with either a) the immunogenic fusion protein of SEQ ID NO: 21 together with the adjuvant aluminum hydroxide (AlOH), b) or control (only the adjuvant aluminum hydroxide).
Fig. 4A-4E show results obtained in a human epithelial cell invasion study using clinical isolates and antisera obtained from immunization using the immunogenic fusion protein of SEQ ID NO: 21.

### DETAILED DESCRIPTION OF THE INVENTION

In this specification, unless otherwise specified, "a" or "an" means "one or more".

Throughout the specification, any and all references are specifically incorporated into this patent application by reference.

As above, a first aspect of the present invention relates to an immunogenic fusion protein capable of eliciting protective immunity against group B Streptococcus, the immunogenic fusion protein comprising or consisting of an amino acid sequence consisting of:
i. a first amino acid sequence part consisting of 170 to 178 amino acids, preferably 174 to 175 amino acids, and having at least 90% sequence identity with the amino acid sequence shown in SEQ ID NO 7;
ii. a second amino acid sequence part consisting of 165 to 174 amino acids, preferably 169 to 170 amino acids, and having at least 90% sequence identity with the amino acid sequence shown in SEQ ID NO 14; and optionally:
iii. a linker amino acid sequence part consisting of 1 to 20 amino acids and separating the first amino acid sequence part from the second amino acid part,
and wherein the immunogenic fusion protein preferably consists of 335 to 372 amino acids, preferably 343 to 353 amino acids, more preferably 343 to 347 amino acids.

The term "immunogenic" is intended to mean having the ability to elicit an immune response. The immunogenic fusion protein of the invention is immunogenic and characterized by its ability to elicit a protective immune response against at least GBS containing the surface proteins of which the N-terminal regions are comprised by the immunogenic fusion protein. Eliciting protective immunity against group B Streptococcus is to be understood as encompassing eliciting protective immunity against group B streptococcus carrying at least one of the surface proteins Rib and AlpC. "Capable" means that the immunogenic fusion protein, if administered in a suitable dose to an individual having an immune system, elicits protective immunity in that individual.

The term "protective immunity" in relation to the present invention refers to the ability of serum antibodies and/or cytotoxic T cell response induced during immunization to protect (partially or totally) against disease caused by an infectious agent, such as a group B Streptococcus. That is, a vertebrate immunized by the vaccines of the invention will experience limited growth and spread of group B Streptococcus. To determine whether protective immunity is induced by a fusion protein or vaccine, techniques well known for a person skilled in the art can be used. For example, to determine whether immunization with a fusion protein or vaccine of the invention induces protective immunity against group B Streptococcus infection, immunized test animals can be challenged with group B Streptococcus and growth and spread of the group B Streptococcus is measured. For example, to determine whether protective immunity is induced, methods in accordance with the methods described in the examples below can be used.

For the purpose of the present invention the term "fusion protein" refers to an assembly of two or more protein regions, or fragments thereof. As stated above, the immunogenic fusion protein comprises (or preferably consists) of an amino acid sequence consisting of at least first and second amino acid sequence parts. These parts are related to the N-terminal regions of the group B Streptococcus surface proteins Rib and AlpC, respectively.

The combination of amino acid sequence parts to obtain a fusion protein can be accomplished by fusing, i.e., joining, the amino acid sequence parts together at one or both ends thereof (covalent coupling, bond, or linking, or conjugation), or by joining together separate nucleic acid molecules encoding the respective amino acid sequence parts, such that a single continuous expression product is produced when the joined nucleic acid molecules are translated.

In a fusion protein, a first and a second amino acid sequence part may be separated by a linker amino acid sequence part.

It is further contemplated within the context of the present invention that a fusion protein can be obtained by cross-linking the amino acid sequence parts or by capturing them on a macromolecular structure.

For the purpose of the present invention the term "protein" refers to a molecular chain of amino acids. A protein is not of a specific length and can, if required, be modified in vivo or in vitro, by, for example, glycosylation, amidation, carboxylation or phosphorylation. Inter alia, peptides, oligopeptides and polypeptides are included within the definition.

The term "N-terminal region" in relation to the present invention refers to an N-terminus region (N) of a protein.

Group B streptococcus strains, also referred herein as GBS, are known and may be isolated from the blood of infected human beings. GBS is the most common cause of neonatal sepsis in the United States and is responsible for about 5000 cases per year.
The denotation "Group B streptococcus" derives from the fact that Streptococci have been divided into immunological groups based upon the presence of specific carbohydrate antigens on their cell surfaces. At present, groups A through O are recognized.

The group B Streptococcus Rib protein, also referred to in this specification as Rib and Rib protein, is a surface protein known in the art, and for example described in WO 9421685. The denotation "Rib" refers to: Resistance to proteases, immunity, and group B. The Rib protein was first isolated from a group B streptococcal strain of serotype III as a distinct 95 kDa protein. Protein Rib is expressed by almost all group B streptococcal strains of the clinically important serotype III, which cause most cases of meningitis, and by some strains of other serotypes such as II. Moreover, Rib is expressed by all strains of a hypervirulent clone of type III. A method has been devised to purify protein Rib and it has been demonstrated that antibodies to this protein protect against lethal infection with strains expressing protein Rib (for further details, such as DNA and protein sequences see WO 9421685).

SEQ ID NO: 7 shows the shorter N-terminal Region of Rib:
MAEVISGSAVTLNTNMTKNVQNGRAYIDLYDVKNGKIDPLQLITLNSPDLK
AQYVIRQGGNYFTQPSELTTVGAASINYTVLKTDGSPHTKPDGQVDIINVSL
TIYNSSALRDKIDEVKKKAEDPKWDEGSRDKVLISLDDIKTDIDNNPKTQSD
IANKITEVTNLEKILVPRIP

Nucleic acid DNA molecules encoding the amino acid sequence of SEQ ID NO: 7 are shown in SEQ ID NOs: 1, 3, 5, where SEQ ID NO: 3 is codon optimized for expression in *E. coli,* and SEQ ID NO: 5 is codon optimized for expression in *Cricetulus griseus* (Chinese Hamster Ovary) cells.

Corresponding nucleic acid RNA molecules encoding the amino acid sequence of SEQ ID NO: 7 are shown in SEQ ID NOs: 2, 4, 6, where SEQ ID NO: 4 is codon optimized for expression in *E. coli,* and SEQ ID NO: 6 is codon optimized for expression in *Cricetulus griseus* (Chinese Hamster Ovary) cells.

The group B Streptococcus AlpC protein, also known as alpha protein, is a group B Streptococcus surface protein known in the art. WO 9410317 describes a conjugate vaccine composition comprising the alpha protein. The native group B Streptococcus AlpC precursor protein as described in WO 9410317 has a molecular weight of 108 kDa. Cleavage of the putative signal sequence of 41 amino acids yields a mature protein of 104 kDa. (Note, however, that the signal sequence was subsequently shown to have a length of 56 amino acid residues: Stalhammar-Carlemalm et al., J Exp Med 177,1593; 1993). The 20 kDa N-terminal region of the AlpC antigen shows no homology to previously described protein sequences and is followed by a series of nine tandem repeating units that make up 74% of the mature protein. Each repeating unit (denoted herein as "R") is identical and consists of 82 amino acids with a molecular mass of about 8500 Daltons, which is encoded by 246 nucleotides. The C-terminal region of the AlpC antigen contains a cell wall anchor domain motif present in a number of gram-positive surface proteins.

SEQ ID NO: 14 shows the shorter N-terminal Region of AlpC:
STIPGSAATLNTSITKNIQNGNAYIDLYDVKLGKIDPLQLIVLEQGFTAKYVF
RQGTKYYGDVSQLQSTGRASLTYNIFGEDGLPHVKTDGQIDIVSVALTIYDS
TTLRDKIEEVRTNANDPKWTEESRTEVLTGLDTIKTDIDNNPKTQTDIDSKIV
EVNELEKLLVLS

Nucleic acid DNA molecules encoding the amino acid sequence of SEQ ID NO: 14 are shown in SEQ ID NOs: 8, 10, 12, where SEQ ID NO: 10 is codon optimized for expression in *E. coli,* and SEQ ID NO: 12 is codon optimized for expression in *Cricetulus griseus* (Chinese Hamster Ovary) cells.

Corresponding nucleic acid RNA molecules encoding the amino acid sequence of SEQ ID NO: 14 are shown in SEQ ID NOs: 9, 11, 13, where SEQ ID NO: 11 is codon optimized for expression in *E. coli,* and SEQ ID NO: 13 is codon optimized for expression in *Cricetulus griseus* (Chinese Hamster Ovary) cells.

The term "sequence identity" indicates a quantitative measure of the degree of homology between two amino acid sequences of equal length or between two nucleotide sequences of equal length. If the two sequences to be compared are not of equal length, they must be aligned to best possible fit. Sequence identity can, for example, be calculated by the BLAST program e.g., the BLASTP program or the BLASTN program (Pearson W. R and D. J. Lipman (1988) PNAS USA 85:2444-2448) (www.ncbl.nlm.nlh.gov/BLAST).
Preferably the immunogenic fusion protein consists of the amino acid sequences.

The first amino acid sequence part should consist of at the most 178 amino acids, such as 170 to 178 amino acids, preferably 174 to 175 amino acids. As noted, SEQ ID NO: 7 contains 175 amino acids. The first M in the sequence is optional, thus resulting in 174 amino acids in these cases.
The first amino acid sequence part has at least 90%, such as at least 95%, such as at least 97%, at least 98%, at least 99%, or 100% sequence identity with the amino acid sequence shown in SEQ ID NO: 7.

The second amino acid sequence part should consist of at the most 174 amino acids, such as 165 to 174 amino acids, preferably 169 to 170 amino acids. As noted, SEQ ID NO: 14 contains 170 amino acids. The first A in the sequence may be optional, thus resulting in 169 amino acids in these cases.
The second amino acid sequence part has at least 90%, such as at least 95%, such as at least 97%, at least 98%, at least 99%, or 100% sequence identity with the amino acid sequence shown in SEQ ID NO: 14.

The linker amino acid sequence part is optional. In other words, the linker amino acid sequence part, if included, may consist of 0 to 20 amino acids. The amino acids in the linker amino acid sequence part may be selected randomly, i.e., to obtain a random coil formation. The linker amino acid sequence part, if present, separates the first amino acid sequence part from the second amino acid part. In other words, the linker amino acid sequence part is provided between the first amino acid sequence part from the second amino acid part.

Either of the first and second amino acid sequence parts may be provided with a leading/trailing amino acid sequence part at the end furthest away from the other of the first and second amino acid sequence parts. The leading/trailing amino acid sequence part may comprise one or more amino acids.

The immunogenic fusion protein preferably consists of 335 to 372 amino acids, preferably 343 to 353 amino acids, more preferably 343 to 347 amino acids.

In a preferred embodiment of the immunogenic fusion protein according to the first aspect of the present invention:
- the first amino acid sequence part comprises, or preferably consists of, amino acids 1-175 or 2-175 of the sequence shown in SEQ ID NO: 7, and
- the second amino acid sequence part comprises, or preferably consists of, amino acids 1-170 or 2-170 of the sequence shown in SEQ DI NO: 14.
SEQ ID NO: 7 contains 175 amino acids. Amino acids 2-175 correspond to the sequence of amino acids in SEQ ID NO: 7 except for the first methionine (M)

In a preferred embodiment of the immunogenic fusion protein according to the first aspect of the present invention, the amino acid sequence comprises the linker amino acid sequence part, and the linker amino acid sequence part consists of 1 to 10 amino acids, preferably 2 to 5 amino acids, more preferably 2 amino acids, and most preferably the amino acid sequence EF.
When the linker amino acid sequence part consists of the amino acids EF (Glutamate-Phenylalanine), then it is preferable that the second amino acid sequence part comprises, or preferably consists of, amino acids 2-170 of the sequence shown in SEQ DI NO: 14.

In a preferred embodiment the immunogenic fusion protein consists of the sequence of amino acids shown in SEQ ID NO: 21, or alternatively, the immunogenic fusion protein consists of the amino acids 2-347 of SEQ ID NO: 21.
As shown in relation to the figures, such an immunogenic fusion protein is shorter than the immunogenic fusion protein disclosed in WO 2008127179 yet has a good effect and is suitable to use in a vaccine against GBS.

SEQ ID NO: 21 corresponds to the amino acid sequence:
MAEVISGSAVTLNTNMTKNVQNGRAYIDLYDVKNGKIDPLQLITLNSPDLK
AQYVIRQGGNYFTQPSELTTVGAASINYTVLKTDGSPHTKPDGQVDIINVSL
TIYNSSALRDKIDEVKKKAEDPKWDEGSRDKVLISLDDIKTDIDNNPKTQSD
IANKITEVTNLEKILVPRIPEFSTIPGSAATLNTSITKNIQNGNAYIDLYDVKL
GKIDPLQLIVLEQGFTAKYVFRQGTKYYGDVSQLQSTGRASLTYNIFGEDGL
PHVKTDGQIDIVSVALTIYDSTTLRDKIEEVRTNANDPKWTEESRTEVLTGL
DTIKTDIDNNPKTQTDIDSKIVEVNELEKLLVLS

In a further embodiment of the immunogenic fusion protein according to the first aspect of the present invention the immunogenic fusion protein is modified by glycosylation, amidation, carboxylation or phosphorylation, or by being conjugated to a capsular polysaccharide or an RSV antigen as described with regard to the third aspect of the present invention further below.
This is advantageous as such fusion proteins may have enhanced immunogenicity. Numerous techniques are available and well known to those of skill in the art which may be used, without undue experimentation, to substantially increase the immunogenicity of the fusion protein. For example, the fusion protein may be modified by coupling to dinitrophenol groups or arsanilic acid, or by denaturation with heat and/or SDS. Alternatively, the fusion protein may be coupled to an immunogenic carrier. For a review of some general considerations in coupling strategies, see Antibodies, A Laboratory Manual, Cold Spring Harbor Laboratory, ed. E. Harlow and D. Lane (1988). Useful immunogenic carriers are well known in the art. Examples of such carriers are keyhole limpet hemocyanin (KLH); albumins such as bovine serum albumin (BSA) and ovalbumin, PPD (purified protein derivative of tuberculin); red blood cells; tetanus toxoid; cholera toxoid; agarose beads; activated carbon; or bentonite.
In a further embodiment the immunogenic fusion protein is conjugated to a capsular polysaccharide, preferably a bacterial polysaccharide, more preferably a group B Streptococcus polysaccharide.
By polysaccharide is meant any linear or branched polymer consisting of monosaccharide residues, usually linked by glycosidic linkages, and thus includes oligosaccharides. Preferably, the polysaccharide will contain between 2 and 50 monosaccharide unites, more preferably between 6 and 30 monosaccharide units. The polysaccharide component may be based on or derived from polysaccharide components of the polysaccharide capsule from many gram-positive and gram-negative bacterial pathogens such as *H. influenzae, N. meningitidis* and *S. pneumoniae.* Other bacteria from which polysaccharide components may be conjugated to the fusion proteins of the present invention include *Staphylococcus aureus, Klebsiella, Pseudomonas, Salmonella typhi, Pseudomonas aeruginosa,* and *Shigella dysenteriae.* Polysaccharide components suitable for use according to this aspect of the present invention include the Hib oligosaccharide, lipopolysaccharide from *Pseudomonas aeruginosa,* lipopolysaccharides from Salmonella, and the O-specific polysaccharide from *Shigella dysenteriae.*
Other polysaccharide components suitable for use in accordance with the present invention will be well-known to those skilled in the art.
Fragments of bacterial capsular polysaccharide may be produced by any suitable method, such as by acid hydrolysis or ultrasonic irradiation. Other methods of preparation of the polysaccharide components will be well known to those of skill in the art.

The polysaccharide is preferably a capsular polysaccharide derived from group B Streptococcus, or their equivalents.
The polysaccharide should preferably be coupled to the fusion protein by a covalent linkage. A particularly preferred method of coupling polysaccharide and protein is by reductive amination. Other methods include: activation of the polysaccharide with cyanogen bromide followed by reaction with adipic acid dihydrazide (spacer) and by conjugation to carboxide groups of carrier protein using soluble carbodiimides; functionalisation of the carrier protein with adipic acid dihydrazide followed by coupling to cyanogen bromide activated polysaccharides, chemical modification of both the carrier protein and the polysaccharide followed by their coupling.
The polysaccharide molecule may be coupled to the fusion protein by a spacer molecule, such as adipic acid. This spacer molecule can be used to facilitate the coupling of fusion protein to polysaccharide. After the coupling reaction has been performed, the conjugate may be purified by diafiltration or other known methods to remove unreacted protein or polysaccharide components.

If the polysaccharide is derived from a bacterial pathogen different from GBS, the conjugate may elicit immunity against two or more pathogens, e.g., multiple types of bacteria. This is a potentially important application of the immunogenic fusion protein.

A second aspect of the present invention concerns a nucleic acid molecule comprising a polynucleotide encoding the immunogenic fusion protein according to the first aspect of the present invention.
Such a nucleic acid molecule can be used in the manufacture of the immunogenic fusion protein directly, e.g., in a cell culture from which the immunogenic fusion protein is separated and obtained, or indirectly, e.g., in a subject in which the immunogenic fusion protein is to be expressed for immunization.
The nucleic acid molecule can e.g., be a DNA molecule or an RNA molecule. The RNA molecule may be a mRNA molecule.

The nucleic acid molecule may comprise, or preferably consist of, a first polynucleotide having at least 90%, such as at least 95%, at least 96%, at least 97%, at least 98% or at least 99%, or 100%, sequence identity to one of SEQ ID NOs: 1-6 fused directly, or via a linker polynucleotide sequence, to a second polynucleotide having at least 90%, such as at least 95%, at least 96%, at least 97%, at least 98% or at least 99%, or 100%, sequence identity to one of SEQ ID NOs: 8-13.
The linker polynucleotide sequence is optional and included if the immunogenic fusion protein encoded by the nucleic acid molecule is to have a linker provided between the first amino acid sequence part and the second amino acid sequence part. If the first polynucleotide is fused directly to the second polynucleotide, then there is no linker polynucleotide sequence present in the nucleic acid molecule. Each of the SEQ ID NOs: 1-6 and the SEQ ID NO:s 8-13 referred to above may be optionally truncated by removing the first three bases coding for the initial methionine (M) in the first amino acid sequence part, and the initial alanine (A) in the second amino acid sequence part of the immunogenic fusion protein encoded by the nucleic acid molecule.
The nucleic acid molecule may optionally comprise one or more stop codons.
The nucleic acid molecule may optionally comprise one or more start codons.
The nucleic acid molecule may be codon optimized for expression in a specific organism, such as *E. coli,* or *Cricetulus griseus* (Chinese Hamster Ovary) cells.

In a preferred embodiment of the nucleic acid molecule according to the second aspect of the present invention, the nucleic acid molecule has at least 90% sequence identity with the sequence of nucleotides shown in one of SEQ ID NOs: 15-20. Preferably the nucleic acid molecule has at least at least 95%, at least 96%, at least 97%, at least 98% or at least 99%, or 100%, sequence identity with the sequence of nucleotides shown in one of SEQ ID NOs: 15-20. Each of the SEQ ID NOs: 15-20 referred to in these preferred embodiments may be optionally truncated by removing the first three bases coding for the initial methionine (M) in the first amino acid sequence part of the immunogenic fusion protein encoded by the nucleic acid molecule. Further, each of the SEQ ID NOs: 15-20 referred to in these preferred embodiments may be optionally truncated by removing the last 1 to 6 bases of the two stop codons. This may for example be used when the nucleic acid molecule is to be used as a DNA or RNA vaccine - transcription and/or translation will cease at the end of the nucleic acid molecule.
The nucleic acid molecule may preferably consist of 1044 (without initial M) to 1047 bases (with initial M).

A third aspect of the present invention concerns a vector comprising the nucleic acid molecule according to the second aspect of the present invention.
A wide variety of expression host/vector combinations may be employed in expressing the nucleic acid molecule. Useful expression vectors for eukaryotic hosts include, for example, vectors comprising expression control sequences from SV40, bovine papilloma virus, adenovirus, adeno-associated virus, cytomegalovirus, and retroviruses. Useful expression vectors for bacterial hosts include bacterial plasmids, such as those from *E. coli,* including pBluescript, pGEX2T, pUC vectors, col E1, pCR1, pBR322, pMB9 and their derivatives, wider host range plasmids, such as RP4, phage DNAs, e.g., the numerous derivatives of phage lambda, e.g., lambda GT10 and lambda GT11, NM989, and other DNA phages, such as M13 and filamentous single stranded DNA phages. Useful expression vectors for yeast cells include the 2.mu. plasmid and derivatives thereof. Useful vectors for insect cells include pVL 941.
In addition, any of a wide variety of expression control sequences may be used in these vectors to express the nucleic acid molecules. Useful expression control sequences include the expression control sequences associated with structural genes of the foregoing expression vectors. Examples of useful expression control sequences include, for example, the early and late promoters of SV40 or adenovirus, the lac system, the trp system, the TAC or TRC system, the T3 and T7 promoters, the major operator and promoter regions of phage lambda, the control regions of fd coat protein, the promoter for 3-phosphoglycerate kinase or other glycolytic enzymes, the promoters of acid phosphatase, e.g., Pho5, the promoters of the yeast alpha-mating system and other constitutive and inducible promoter sequences known to control the expression of genes of prokaryotic or eukaryotic cells or their viruses, and various combinations thereof.

A fourth aspect of the present invention concerns a host cell expressing the immunogenic fusion protein according to the first aspect of the present invention or comprising the nucleic acid molecule according to the second aspect of the presentation invention or the vector according to the third aspect of the present invention.
The host cell may be a gram-negative bacterial cell, gram-positive bacterial cell, yeast cell, insect cell, animal cell, African green monkey cell, human cell, or plant cell. The host cell may be selected from the group consisting of *E. coli, Pseudomonas, Bacillus, Streptomyces, aspergillus, lactobacillus, shigella, salmonella, listeria, streptococcus, staphylococcus* and fungi. *E. coli* is however preferred.
Additionally, insect cells such as *Spodoptera frugiperda* (SF9), animal cells such as CHO and mouse cells, African green monkey cells such as COS 1, COS 7, BSC 1, BSC 40, and BMT 10, human cells, and plant cells in tissue culture, may be used. Preferred host organisms include bacteria such as *E. coli* and *B. subtilis,* and mammalian cells in tissue culture.

When a host cell is used to produce the immunogenic fusion protein, or used to produce the first and second amino acid sequence parts thereof, or used to produce the linker amino acid sequence part, then the produced amino acid sequence parts may be isolated from the microbial culture or cell culture and purified using any of a variety of conventional methods including: liquid chromatography such as normal or reversed phase, using HPLC, FPLC and the like; affinity chromatography (such as with inorganic ligands or monoclonal antibodies); ion exchange chromatography, size exclusion chromatography; immobilized metal chelate chromatography; gel electrophoresis; tangential flow filtration and the like. One of skill in the art may select the most appropriate isolation and purification techniques without departing from the scope of this invention.

In addition, the immunogenic fusion protein, the first and second amino acid sequence parts thereof, or the linker amino acid sequence part, may be generated by any of several chemical techniques. For example, they may be prepared using the solid-phase synthetic technique or they may be prepared by synthesis in solution. A

A fifth aspect of the present invention concerns a vaccine comprising one or more of the immunogenic fusion protein according to the first aspect of the present invention, the nucleic acid molecule according to the second aspect of the present invention, the vector according to the third aspect of the present invention, or the host cell according to the fourth aspect of the present invention.
The amount of each of the immunogenic fusion, the nucleic acid molecule, the vector, and/or the host cell is preferably a pharmaceutically effective amount, or an amount capable of eliciting protective immunity against group B Streptococcus, or an amount sufficient to elicit an immune response. Further, the amount may be such as to be sufficient to reduce and most preferably prevent a clinically significant deficit in the activity and response of the subject receiving the vaccine. Alternatively, the amount is sufficient to cause an improvement in a clinically significant condition in a host or subject.
As is appreciated by those skilled in the art, the amount of a compound may vary depending on its specific activity. Suitable dosage amounts may contain a predetermined quantity of active composition calculated to produce the desired therapeutic effect in association with the required diluents; i.e., carrier, or additive. Further, the dosage to be administered will vary depending on the active principle or principles to be used, the age, weight, etc. of the individual to be treated.
The vaccine is preferably capable of eliciting protective immunity against group B Streptococcus.

As noted in the figures, the immunogenic fusion protein elicits an immune response when administered to subjects. Such an immune response can also be obtained, indirectly, by administering the nucleic acid molecule either as a DNA nucleic acid molecule for later transcription and translation for expressing the immunogenic fusion protein, or as an RNA or mRNA nucleic acid molecule for translation and expression of the immunogenic fusion protein. The nucleic acid molecule may be provided in the vector for increased transcriptional and/or translational control, and the vector can in turn be provided in the host cell for further controlling and/or increasing the expression of the immunogenic fusion protein.

The vaccine may further comprise a pharmaceutically acceptable vehicle, and optionally an adjuvant. The term "pharmaceutical acceptable vehicle" is intended to mean any suitable acceptable excipient, adjuvants, carrier, diluent commonly used in pharmaceutical formulations.
The vaccine may encompass a vaccine composition.
The vaccine may additionally comprise other pharmacologically acceptable ingredients such as salts, buffers, immunoactive components, adjuvants (AlOH), wetting agents, emulsifying and suspending agents, or sweetening, flavouring, perfuming agents, or other substances which are desirable for improving the efficacy of the composition. A composition is said to be "pharmacologically acceptable" if its administration can be tolerated by a recipient individual.

Adjuvants are substances that can be used to specifically augment a specific immune response. Normally, the adjuvant and the composition are mixed prior to presentation to the immune system, or presented separately, but into the same site of the animal or human being immunized. Adjuvants can be loosely divided into several groups based upon their composition. These groups include oil adjuvants (for example, Freund's complete and incomplete), mineral salts for example, AlK (SO₄)₂, AlNa (SO₄)₂, AlNH₄ (SO₄), AlOH, silica, kaolin, and carbon), polynucleotides (for example, poly IC and poly AU acids), and certain natural substances (for example, wax D from *Mycobacterium tuberculosis,* as well as substances found in *Corynebacterium parvum,* or *Bordetella pertussis,* and members of the genus *Brucella.*
Alhydrogel (e.g., Aluminum hydroxide, AlOH) is an especially effective adjuvant for the immunogenic fusion protein according to the first aspect of the present invention and therefore especially useful in the vaccine according to the fifth aspect of the present invention.

Methods for the preparation and formulation of vaccines and vaccine compositions are well known to those skilled in the art. The choice of ingredients will for instance vary depending on the administration route of the composition. For example, compositions for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Carriers or occlusive dressings can be used to increase skin permeability and enhance antigen absorption. Liquid dosage forms for oral administration may generally comprise a liposome solution containing the liquid dosage form. Suitable forms for suspending liposomes include emulsions, suspensions, solutions, syrups, and elixirs containing inert diluents commonly used in the art, such as purified water.

The vaccine may be administered to humans or animals, including mammals and birds, such as rodents (mouse, rat, guinea pig, or rabbit); birds (turkey, hen or chicken); other farm animals (cow, horse, pig or piglet); pets (dog, cat and other pets); and humans. While many animals may be treated with the vaccine of the invention, a preferred individual for treatment is a human or commercially valuable animal and livestock such as fish, e.g., Tilapia, and camels.

The vaccine can be administered to an individual according to methods known in the art. Such methods comprise application e.g., parenterally, such as through all routes of injection into or through the skin: e.g., intramuscular, intravenous, intraperitoneal, intradermal, mucosal, submucosal, or subcutaneous. Also, they may be applied by topical application as a drop, spray, gel or ointment to the mucosal epithelium of the eye, nose, mouth, anus, or vagina, or onto the epidermis of the outer skin at any part of the body. Other possible routes of application are by spray, aerosol, or powder application through inhalation via the respiratory tract. In this last case the particle size that is used will determine how deep the particles will penetrate into the respiratory tract. Alternatively, application can be via the alimentary route, by combining with the food, feed or drinking water e.g., as a powder, a liquid, or tablet, or by administration directly into the mouth as a: liquid, a gel, a tablet, or a capsule, or to the anus as a suppository.

Many different techniques exist for the timing of the immunizations. It is possible to administer the vaccine or the components a-d more than once to increase the levels and diversities of expression of the immunoglobulin repertoire expressed by the immunized animal. Typically, if multiple immunizations are given, they may be given a week to three months apart, such as two weeks to two months apart, such as one to two months apart, or four weeks apart.
Additionally, one or maybe more booster injections of the vaccine or the components a-d may be administered. Preferably, booster injections may be administered at about 1 and 6 months after the initial injection or injections.

The vaccine according to the fifth aspect of the present invention may preferably comprise one or more additional immune active components. The additional immunoactive component may be an antigen, an immune enhancing substance, and/or a vaccine; either of these may comprise an adjuvant.

Accordingly, in a preferred embodiment of the nucleic acid molecule according to the second aspect of the present invention, the vaccine additionally comprises one or more of:
a) a further immunogenic fusion protein comprising or consisting of an amino acid sequence having at least 90%, such as 95, 96, 97, 98 or 99 % sequence identity with the amino acid sequence shown in SEQ ID NO: 42;
b) a further nucleic acid molecule encoding the further immunogenic fusion protein, the further nucleic acid molecule preferably having at least 90% sequence identity with the sequence of nucleotides shown in one of SEQ ID NOs: 36-41;
c) a further vector comprising the further nucleic acid molecule; or
d) a further host cell expressing the further immunogenic fusion protein, or comprising the further vector.
As will be discussed further below, the further immunogenic fusion protein is derived from the N-terminal regions of the GBS surface proteins Alp1 and Alp2/3, as also disclosed in WO 2017/068112. As shown in the figures and discussed below, there is a synergistic effect when using a vaccine comprising both the immunogenic fusion protein and the further immunogenic fusion protein.

Further, by including one or more additional component of a-d, the vaccine can provide a broader coverage of protection against all Group B Streptococcus strains. Similar to as described above for the immunogenic fusion protein, the further immunogenic fusion protein may be expressed indirectly by the further nucleic acid molecule, further vector, and the further host cell.

The amount of each of a-d should preferably be a pharmaceutically effective amount, or an amount capable of eliciting protective immunity against group B Streptococcus, or an amount sufficient to elicit an immune response, or at least an amount sufficient to increase the general immune response to the vaccine.

As noted above, the further immunogenic fusion protein has at least 90%, such as 95, 96, 97, 98 or 99 % sequence identity with the amino acid sequence shown in SEQ ID NO: 42, which SEQ ID NO: 42 represents the following amino acid sequence:

MAEVISGSAATLNSALVKNVSGGKAYIDIYDVKNGKIDPLNLIVLTPSNYSA
NYYIKQGGRIFTSVNQLQTPGTATITYNILDENGNPYTKSDGQIDIVSLVTTV
YDTTELRNNINKVIENANDPKWSDDSRKDVLSKIEVIKNDIDNNPKTQSDID
NKIVEVNELEKLLVLPEFSTIPGSAATLNTSITKNIQNGNAYIDLYDVKNGLI
DPQNLIVLNPSSYSANYYIKQGAKYYSNPSEITTTGSATITFNILDETGNPHK
KADGQIDIVSVNLTIYDSTALRNRIDEVINNANDPKWSDGSRDEVLTGLEKI
KKDIDNNPKTQIDIDNKINEVNEIEKLLVVSL

Nucleic acid DNA molecules encoding the amino acid sequence of SEQ ID NO: 42, i.e., as encompassed by (b) are shown in SEQ ID NOs: 36, 38, and 40, where SEQ ID NO: 38 is codon optimized for expression in *E. coli,* and SEQ ID NO: 40 is codon optimized for expression in *Cricetulus griseus* (Chinese Hamster Ovary) cells.

Corresponding nucleic acid RNA molecules encoding the amino acid sequence of SEQ ID NO: 42 are shown in SEQ ID NOs: 37, 39, and 41, where SEQ ID NO: 39 is codon optimized for expression in *E. coli,* and SEQ ID NO: 41 is codon optimized for expression in *Cricetulus griseus* (Chinese Hamster Ovary) cells.

As mentioned above, the further immunogenic fusion protein is derived from the N-terminal regions of the GBS surface proteins Alp1 and Alp2/3.

The Alp1 protein is also known as epsilon protein and is a group B streptococcal alpha-protein-like protein.
The amino acid sequence of the N-terminal region of Alp1 is given by SEQ ID NO: 28:

MAEVISGSAATLNSALVKNVSGGKAYIDIYDVKNGKIDPLNLIVLTPSNYSA
NYYIKQGGRIFTSVNQLQTPGTATITYNILDENGNPYTKSDGQIDIVSLVTTV
YDTTELRNNINKVIENANDPKWSDDSRKDVLSKIEVIKNDIDNNPKTQSDID
NKIVEVNELEKLLVLP

Nucleic acid DNA molecules encoding the amino acid sequence of SEQ ID NO: 28 are shown in SEQ ID NOs: 22, 24, and 26, where SEQ ID NO: 24 is codon optimized for expression in *E. coli,* and SEQ ID NO: 26 is codon optimized for expression in *Cricetulus griseus* (Chinese Hamster Ovary) cells.

Corresponding nucleic acid RNA molecules encoding the amino acid sequence of SEQ ID NO: 28 are shown in SEQ ID NOs: 23, 25, and 27, where SEQ ID NO: 25 is codon optimized for expression in *E. coli,* and SEQ ID NO: 27 is codon optimized for expression in *Cricetulus griseus* (Chinese Hamster Ovary) cells.

The Alp2 protein is another alpha-protein-like-protein. Like the other members of the family, the Alp2 protein has an N-terminal domain and several repeated domains towards the C-terminus. The N-terminal region of Alp2 and Alp3 are identical. Alp3 is another alpha-protein-like-protein, also known as R28. It is very similar to the R28 protein also found in *S. pyrogenes.*
The amino acid sequence of the N-terminal region of Alp2 is given by SEQ ID NO: 35:

STIPGSAATLNTSITKNIQNGNAYIDLYDVKNGLIDPQNLIVLNPSSYSANYYI
KQGAKYYSNPSEITTTGSATITFNILDETGNPHKKADGQIDIVSVNLTIYDST
ALRNRIDEVINNANDPKWSDGSRDEVLTGLEKIKKDIDNNPKTQIDIDNKIN
EVNEIEKLLVVSL

Nucleic acid DNA molecules encoding the amino acid sequence of SEQ ID NO: 35 are shown in SEQ ID NOs: 29, 31, and 33, where SEQ ID NO: 31 is codon optimized for expression in *E. coli,* and SEQ ID NO: 33 is codon optimized for expression in *Cricetulus griseus* (Chinese Hamster Ovary) cells.

Corresponding nucleic acid RNA molecules encoding the amino acid sequence of SEQ ID NO: 35 are shown in SEQ ID NOs: 30, 32, and 34, where SEQ ID NO: 32 is codon optimized for expression in *E. coli,* and SEQ ID NO: 34 is codon optimized for expression in *Cricetulus griseus* (Chinese Hamster Ovary) cells.

SEQ ID NO: 42 thus correspond to SEQ ID NO: 28 (N-terminal of Alp1) fused via a linker amino acid sequence part consisting of the amino acids EF to SEQ ID NO: 35 (N-terminal of Alp2/3).
The second immunogenic fusion protein may thus comprise or consist of an amino acid sequence consisting of:
i. a further first amino acid sequence part having at least 90% sequence identity with the amino acid sequence shown in SEQ ID NO 28;
ii. a further second amino acid sequence part having at least 90% sequence identity with the amino acid sequence shown in SEQ ID NO 35; and optionally:
iii. a further linker amino acid sequence part consisting of 1 to 20 amino acids and separating the further first amino acid sequence part from the further second amino acid part,

In each of SEQ ID NO: 28 and 42, the initial M may be optional. Accordingly, in a preferred embodiment of the vaccine according to the fifth aspect of the present invention the vaccine comprises:
a) the immunogenic fusion protein according to the first aspect of the present invention, wherein the immunogenic fusion protein consists of the sequence of amino acids shown in SEQ ID NO: 21, or alternatively, wherein the immunogenic fusion protein consists of the amino acids 2-347 of SEQ ID NO: 21; and
b) a further immunogenic fusion protein comprising, preferably consisting of, the sequence of amino acids shown in SEQ ID NO: 42, or alternatively, comprising, preferably consisting of, the amino acids 2-347 of SEQ ID NO: 42.
In these embodiments there are preferably other no other proteins or amino acids sequences, different from the specified immunogenic fusion protein and the specified further immunogenic fusion protein, in the vaccine.

In a preferred embodiment of the vaccine according to the fifth aspect of the present invention the vaccine further comprises aluminum hydroxide as an adjuvant. In these embodiments the vaccine is preferably a pharmaceutical composition and further comprises a pharmaceutically acceptable excipient, diluent or carrier.

A sixth aspect of the present invention concerns the immunogenic fusion protein according to the first aspect of the present invention, the nucleic acid molecule according to the second aspect of the present invention, the vector according to the third aspect of the present invention, the host cell according to fourth aspect of the present invention, or the vaccine according to the fifth aspect of the present invention, for use in i) vaccination against group B Streptococcus infection, or ii) treating a group B Streptococcus infection.
The immunogenic fusion protein, the nucleic acid molecule, the vector, the host cell, or the vaccine may be used for such vaccination or treating by administering the immunogenic fusion protein, the nucleic acid molecule, the vector, the host cell, or the vaccine to a subject in need thereof.

A corresponding seventh aspect of the present invention concerns a method of i) vaccination against group B Streptococcus infection, or ii) treating a group B Streptococcus infection, by administrating the immunogenic fusion protein according to the first aspect of the present invention, the nucleic acid molecule according to the second aspect of the present invention, the vector according to the third aspect of the present invention, the host cell according to fourth aspect of the present invention, or the vaccine according to the fifth aspect of the present invention, to a subject in need thereof.

A corresponding eight aspect of the present invention concerns the use of the immunogenic fusion protein according to the first aspect of the present invention, the nucleic acid molecule according to the second aspect of the present invention, the vector according to the third aspect of the present invention, the host cell according to fourth aspect of the present invention for manufacturing a pharmaceutical composition or medicament, such as a vaccine, for i) vaccination against group B Streptococcus infection, or ii) treating a group B Streptococcus infection

The immunogenic fusion protein according to the first aspect of the present invention, the nucleic acid molecule according to the second aspect of the present invention, the vector according to the third aspect of the present invention, the host cell according to fourth aspect of the present invention, or the vaccine according to the fifth aspect of the present invention may thus be used in a method of preventing or treating an infection caused by a group B Streptococcus.

Maternal immunoprophylaxis with a vaccine, for protecting against infection to group B Streptococcus both in the mother and in the young infant, has long been proposed as a potential route.
Thus, in preferred embodiments of the sixth, seventh and eight aspects of the present invention, the immunogenic fusion protein according to the first aspect of the present invention, the nucleic acid molecule according to the second aspect of the present invention, the vector according to the third aspect of the present invention, the host cell according to fourth aspect of the present invention, or the vaccine according to the fifth aspect of the present invention is administered to a female human subject.
The administration is thus capable of conferring immunity to GBS infection to an unborn offspring of the human female. Further, the administration may also be made to a male if the male is older.
According to this embodiment, the immunogenic fusion protein, the nucleic acid molecule, the vector, the host cell, or the vaccine is administered to a non-pregnant female or to a pregnant female, under conditions of time and amount sufficient to cause the production of antibodies which serve to protect both the female and a fetus or newborn (via passive transfer of antibodies across the placenta).

The terms "preventing or treating" in its various grammatical forms in relation to the present invention refer to preventing, curing, reversing, attenuating, alleviating, ameliorating, inhibiting, minimizing, suppressing, or halting (1) the deleterious effects of a disorder associated with group B Streptococcus infection, (2) disorder progression, or (3) disorder causative agent (group B Streptococcus). Further, the terms "preventing or treating" are contemplated to include the creation of total or partial immunity of the individual to group B Streptococcus infection.

A ninth aspect of the present invention concerns a method for preventing or treating an infection caused by a group B Streptococcus which comprises administering to an individual in need thereof an effective amount of antibodies elicited from the exposure of a second individual to the immunogenic fusion protein, the nucleic acid molecule, the vector, the host cell, or the vaccine according to the first to fifth aspects of the present invention.

According to this aspect, resistance or immunity to group B Streptococcus is conferred to the individual by passive immunization, i.e., the elicited antisera is recovered and directly provided to a recipient suspected of having an infection caused by a group B Streptococcus. It is contemplated that such antisera could be administered to a pregnant female (at or prior to parturition), under conditions of time and amount sufficient so that the antisera would serve to protect either the fetus or newborn (via passive incorporation of the antibodies across the placenta).

The vaccine or antisera of the present invention may, thus, be provided either prior to the onset of infection (so as to prevent or attenuate an anticipated infection) or after the initiation of an actual infection.

A tenth aspect of the present invention concerns a method of producing an immunogenic fusion protein comprising the steps of
- providing a host cell according to the fourth aspect of the present invention,
- multiplying the host cell,
- purifying the immunogenic fusion protein according to the first aspect of the present invention, and
- obtaining the immunogenic fusion protein.

Alternatively, the immunogenic fusion protein can be produced in a cell-free expression system. Such systems comprise all essential factors for expression from an appropriate recombinant nucleic acid, operably linked to a promoter that will function in that particular system.

### Example 1 - immunization of healthy women

Adult healthy women were immunized with an immunogenic fusion protein consisting of SEQ ID NO: 21 alone, or together with a further immunogenic fusion protein according to SEQ ID NO: 42, and with, or without, the adjuvant aluminum hydroxide.

Fig. 1 shows IgG fold increase obtained 29 days after immunization using one dose of either an immunogenic fusion protein according to the first aspect of the present invention alone, or in combination with a further immunogenic fusion protein as encompassed by the vaccine according to the fifth aspect of the present invention, at different dosages with or without the adjuvant aluminum hydroxide.
In Fig. 1, the first 7 results were obtained by administering an immunogenic fusion protein consisting of SEQ ID NO: 21 alone or together with the adjuvant aluminum hydroxide (0.5 mg).
As noted from Fig. 1, the use of Aluminum hydroxide is beneficial as it boosts the IgG fold increase of a 10 µg dose to a value similar to the IgG fold increase of a 50 µg dose. Similarly, a 50 µg dose together with aluminum hydroxide provides similar fold increase to a 250 µg dose.
Fig. 1 further shows that, when a vaccine comprising aluminum hydroxide and both an immunogenic fusion protein according to SEQ ID NO: 21 and a further immunogenic fusion protein according to SEQ ID NO: 42 is used, then doses of 25 µg of each of the immunogenic fusion proteins provide the same or higher fold increase compared to a dosage of 250 µg of the immunogenic fusion protein according to SEQ ID NO: 21. Using aluminum hydroxide and 50 µg of each of the immunogenic fusion proteins provided the highest fold increase at day 29.
The number of subjects (n) for each experiment is given in the figure.

Fig. 2 shows IgG fold increase obtained 57 days from immunization using a first dose and a second dose of either an immunogenic fusion protein according to the first aspect of the present invention alone, or in combination with a further immunogenic fusion protein as encompassed by the vaccine according to the fifth aspect of the present invention, at different dosages with or without an adjuvant. In Fig. 2, the first 7 experiments are obtained by administering an immunogenic fusion protein consisting of SEQ ID NO: 21 alone or together with the adjuvant aluminum hydroxide (0.5 mg).
As noted from Fig. 2, the two doses of the immunogenic fusion protein consisting of SEQ ID NO: 21 at 50 µg and 100 µg together with Aluminum hydroxide provide a fold increase of about 100. However, even higher fold increases are seen when a vaccine comprising aluminum hydroxide and both an immunogenic fusion protein according to SEQ ID NO: 21 and a further immunogenic fusion protein according to SEQ ID NO: 42 is used, even though the dosage of each of the immunogenic fusion proteins is only 25 µg or 50 µg.
The number of subjects (n) for each experiment is given in the figure.

### Example 2 - immunization of female mice to protect pups

Fig. 3 shows survival of mice pups following neonatal bacterial infection with group B Streptococcus strain BM110 (8 x 10³ cfu). The pups were born from female mice having been immunized with either a) the immunogenic fusion protein of SEQ ID NO: 21 together with the adjuvant aluminum hydroxide (AlOH), b) or control (only the adjuvant aluminum hydroxide). As seen in the Fig. 3, mice pups born from mothers having been immunized with the immunogenic fusion protein according to SEQ ID NO: 21 (dashed line) had significantly higher survival rate throughout the experiment (0 - 48 hours post infection) than the mice pups born from mothers receiving the control (solid line). Accordingly, the immunogenic fusion protein of SEQ ID NO: 21, when administered to female mice, provided significant protection against GBS infection and death for mice pups born form those female mice. The immunogenic fusion protein thus provided a protective effect against GBS infection and death similar to as reported for mice in Panel C and E of Figure 2 of Lindahl et al, Nonimmunodominant Regions Are Effective As Building Blocks In A Streptococcal Fusion Protein Vaccine, Cell Host & Microbe 2, 427-434, December 2007.

### Example 3 - epithelial cell invasion study

Figs. 4A-4E show results obtained in a human epithelial cell invasion study using clinical isolates and antisera obtained from immunization using the immunogenic fusion of SEQ ID NO: 21. The respective bars show the relative percentage of epithelial cell invasion for the control (no serum), preimmune stage at day 0 (D0) and post immune stage at day 57 (D57). A good effect on inhibiting invasion of endothelial cells is shown. The immunogenic fusion protein thus provided an inhibitory effect on invasion of epithelial cells similar to as reported in Panel B of Figure 3 of Lindahl et al, Nonimmunodominant Regions Are Effective As Building Blocks In A Streptococcal Fusion Protein Vaccine, Cell Host & Microbe 2, 427-434, December 2007.

Accordingly, Figs 1 and 2 show that it is advantageous to administer the immunogenic fusion protein together with the further fusion protein, and that is also advantageous to use an adjuvant, e.g., aluminum hydroxide.
However, the immunogenic fusion protein elicited an immune response, e.g., IgG fold increase, also at low dosages (10 µg) and without adjuvant.

Further, Figs 3 and 4A-4E show that the immunogenic fusion protein provides effects similar to those seen for the immunogenic fusion protein disclosed in Lindahl et al, Nonimmunodominant Regions Are Effective As Building Blocks In A Streptococcal Fusion Protein Vaccine, Cell Host & Microbe 2, 427-434, December 2007 and WO 2008127179.

The dosage of each of the immunogenic fusion protein, and the further immunogenic fusion protein if administered, for humans may be in the range of 10 µg to 250 µg, such as from 10 µg to 100 µg, such as from 15 µg to 75 µg, preferably 25 µg to 75 µg, such as 25 µg to 50 µg.

The preferred dosage of each of the immunogenic fusion protein, and the further immunogenic fusion protein if administered, for humans, in the presence of an adjuvant such as aluminum hydroxide, is therefore within the range of 10 to 250 µg, preferably 10 to 150 µg, preferably 25 to 100 µg or 25 to 75 µg.

If the immunogenic fusion protein is administered together with the further immunogenic fusion protein, and in the presence of an adjuvant such as aluminum hydroxide, then the dosage of each fusion protein is preferably in the range of 15 µg to 75 µg, preferably 25 µg to 75 µg, such as 25 µg to 50 µg.

In the absence of an adjuvant such as aluminum hydroxide, then the dosage of each of the immunogenic fusion protein, and the further immunogenic fusion protein if administered, for humans, would be 10 to 1000 µg, preferably 50 to 500 µg, or preferably 100 to 250 µg.

If the immunogenic fusion protein, and the further immunogenic fusion protein if administered, are to administered indirectly by administering corresponding nucleic acid molecules, vectors or host cells, then the dosage of the corresponding nucleic acid molecules, vectors or host cells is preferably selected so as to obtain a similar immune response to what would have been obtained if the immunogenic fusion protein, and the further immunogenic fusion protein if administered, were administered directly to the subject.

Preferably the dosage of each of the immunogenic fusion protein, and the further immunogenic fusion protein if administered, is repeated. Accordingly, the immunogenic fusion protein, and the further immunogenic fusion protein if administered, are administered in a first dose, and repeated as a second dose some time after the first dose. The first dose and the second dose may be from a week to three months apart, such as two weeks to two months apart, such as one to two months apart, or four weeks apart.

## Claims

**1.** An immunogenic fusion protein capable of eliciting protective immunity against group B Streptococcus, the immunogenic fusion protein comprising or consisting of an amino acid sequence consisting of:
i. a first amino acid sequence part consisting of 170 to 178 amino acids, preferably 174 to 175 amino acids, and having at least 90% sequence identity with the amino acid sequence shown in SEQ ID NO: 7;
ii. a second amino acid sequence part consisting of 165 to 174 amino acids, preferably 169 to 170 amino acids, and having at least 90% sequence identity with the amino acid sequence shown in SEQ ID NO: 14; and optionally:
iii. a linker amino acid sequence part consisting of 1 to 20 amino acids and separating the first amino acid sequence part from the second amino acid part,
and wherein the immunogenic fusion protein preferably consists of 335 to 372 amino acids, preferably 343 to 353 amino acids, more preferably 343 to 347 amino acids.

**2.** The immunogenic fusion protein according to claim 1, wherein:
- the first amino acid sequence part comprises, or preferably consists of, amino acids 1-175 or 2-175 of the sequence shown in SEQ ID NO: 7, and
- the second amino acid sequence part comprises, or preferably consists of, amino acids 1-170 or 2-170 of the sequence shown in SEQ DI NO: 14.

**3.** The immunogenic fusion protein according to any preceding claim, wherein the amino acid sequence comprises the linker amino acid sequence part, and wherein the linker amino acid sequence part consists of 1 to 10 amino acids, preferably 2 to 5 amino acids, more preferably 2 amino acids, and most preferably the amino acid sequence EF.

**4.** The immunogenic fusion protein according to any preceding claim, wherein the immunogenic fusion protein consists of the sequence of amino acids shown in SEQ ID NO: 21, or alternatively, wherein the immunogenic fusion protein consists of the amino acids 2-347 of SEQ ID NO: 21.

**5.** A nucleic acid molecule comprising a polynucleotide encoding the immunogenic fusion protein according to any of the proceeding claims.

**6.** The nucleic acid molecule according to claim 5, wherein the nucleic acid molecule has at least 90% sequence identity with the sequence of nucleotides shown in one of SEQ ID NOs: 15-20.

**7.** A vector comprising the nucleic acid molecule according to any of the claims 5-6.

**8.** A host cell expressing the immunogenic fusion protein according to any of the claims 1-4 or comprising the nucleic acid molecule according to any of the claims 5-6 or the vector according to claim 7.

**9.** A vaccine comprising one or more of the immunogenic fusion protein according to any of the claims 1-4, the nucleic acid molecule according to any of the claim 5-6, the vector according to claim 7, or the host cell according to claim 8.

**10.** The vaccine according to claim 9, additionally comprising one or more of:
a) a further immunogenic fusion protein comprising or consisting of an amino acid sequence having at least 90%, such as 95, 96, 97, 98 or 99 % sequence identity with the amino acid sequence shown in SEQ ID NO: 42;
b) a further nucleic acid molecule encoding the further immunogenic fusion protein, the further nucleic acid molecule preferably having at least 90% sequence identity with the sequence of nucleotides shown in one of SEQ ID NOs: 36-41;
c) a further vector comprising the further nucleic acid molecule; or
d) a further host cell expressing the further immunogenic fusion protein, or comprising the further vector.

**12.** The vaccine according to claim 11, wherein the vaccine comprises:
a) the immunogenic fusion protein according to claim 4; and
b) a further immunogenic fusion protein comprising, preferably consisting of, the sequence of amino acids shown in SEQ ID NO: 42, or alternatively comprising, preferably consisting of, the amino acids 2-347 of SEQ ID NO: 42.

**13.** The vaccine according to any of the claim 9-12, further comprising aluminum hydroxide as an adjuvant.

**14.** The immunogenic fusion protein according to any of the claims 1-4, the nucleic acid molecule according to any of the claim 5-7, the vector according to claim 8, the host cell according to claim 9, or the vaccine according to any of the claims 10-13, for use in i) vaccination against group B Streptococcus infection, or ii) treating a group B Streptococcus infection.

**15.** The immunogenic fusion protein according to any of the claims 1-4, the nucleic acid molecule according to any of the claim 5-7, the vector according to claim 8, the host cell according to claim 9, or the vaccine according to any of the claims 10-13, for use according to claim 13, wherein the immunogenic fusion protein according to any of the claims 1-4, the nucleic acid molecule according to any of the claim 5-7, the vector according to claim 8, the host cell according to claim 9, or the vaccine according to any of the claims 10-12, is administered to a female human subject.


SEQUENCE LISTING

<110>  MinervaX Aps

<120>  IMMUNOGENIC FUSION PROTEIN

<130>  P30709EP

<160>  42

<170>  PatentIn version 3.5

<210>  1
<211>  525
<212>  DNA
<213>  Streptococcus sp.

<400>  1
atggctgaag taatttcagg aagtgctgtt acgttaaaca caaatatgac taaaaatgtt      60

cagaatggta gagcatatat agatttatat gatgtgaaaa atgggaaaat agatccatta     120

caattaatta cgttaaattc acctgattta aaagctcagt atgtcattag gcaaggcggc     180

aattatttca cacaaccttc tgaattgact actgttggtg cagctagtat taattataca     240

gtattgaaga cagatggaag tcctcatacg aagcctgatg gacaagtgga tattataaac     300

gtttcattga ctatttacaa ttcttcagct ttgagagata aaatagatga agttaaaaag     360

aaagcggaag accctaaatg ggacgaggga agtcgcgata aagttttgat aagtttagat     420

gatatcaaaa cagatattga taataatcct aagacgcaat cagacattgc caataaaata     480

actgaagtta ctaatttaga aaaaatacta gtacctcgaa tccca                     525

<210>  2
<211>  525
<212>  RNA
<213>  Streptococcus sp.

<400>  2
auggcugaag uaauuucagg aagugcuguu acguuaaaca caaauaugac uaaaaauguu      60

cagaauggua gagcauauau agauuuauau gaugugaaaa augggaaaau agauccauua     120

caauuaauua cguuaaauuc accugauuua aaagcucagu augucauuag gcaaggcggc     180

aauuauuuca cacaaccuuc ugaauugacu acuguuggug cagcuaguau uaauuauaca     240

guauugaaga cagauggaag uccucauacg aagccugaug gacaagugga uauuauaaac     300

guuucauuga cuauuuacaa uucuucagcu uugagagaua aaauagauga aguuaaaaag     360

aaagcggaag acccuaaaug ggacgaggga agucgcgaua aaguuuugau aaguuuagau     420

gauaucaaaa cagauauuga uaauaauccu aagacgcaau cagacauugc caauaaaaua     480

acugaaguua cuaauuuaga aaaaauacua guaccucgaa uccca                     525

<210>  3

<211>   525
<212>   DNA
<213>   Streptococcus sp.

<400>   3
atggccgaag ttattagcgg tagcgcagtt accctgaata ccaatatgac caaaaatgtg      60

cagaatggcc gtgcctatat tgatctgtat gatgtgaaaa atggcaaaat cgatccgctg     120

cagctgatta ccctgaattc tccggatctg aaagcacagt atgttattcg tcagggtggt     180

aactatttta cccagccgag cgaactgacc accgttggtg cagcaagcat taactatacc     240

gttctgaaaa ccgatggtag tccgcatacc aaaccggatg gtcaggttga tattatcaat     300

gttagcctga ccatctataa tagcagcgca ctgcgtgata aaattgatga agtgaaaaag     360

aaagccgaag atccgaaatg ggatgaaggt agccgtgata aagttctgat tagcctggat     420

gatatcaaaa ccgatattga taacaacccg aaaacccaga gcgatattgc aaacaaaatt     480

accgaagtga ccaatctgga aaaaattctg gttccgcgta ttccg                     525


<210>   4
<211>   525
<212>   RNA
<213>   Streptococcus sp.

<400>   4
auggccgaag uuauuagcgg uagcgcaguu acccugaaua ccaauaugac caaaaaugug      60

cagaauggcc gugccuauau ugaucuguau gaugugaaaa auggcaaaau cgauccgcug     120

cagcugauua cccugaauuc uccggaucug aaagcacagu auguuauucg ucaggguggu     180

aacuauuuua cccagccgag cgaacugacc accguuggug cagcaagcau uaacuauacc     240

guucugaaaa ccgaugguag uccgcauacc aaaccggaug gucagguuga uauuaucaau     300

guuagccuga ccaucuauaa uagcagcgca cugcgugaua aaauugauga agugaaaaag     360

aaagccgaag auccgaaaug ggaugaaggu agccgugaua aaguucugau uagccuggau     420

gauaucaaaa ccgauauuga uaacaacccg aaaacccaga gcgauauugc aaacaaaauu     480

accgaaguga ccaaucugga aaaaauucug guuccgcgua uuccg                     525


<210>   5
<211>   525
<212>   DNA
<213>   Streptococcus sp.

<400>   5
atggccgaag tgatctccgg ctctgctgtg accctgaaca ccaacatgac caagaacgtg      60

cagaacggca gagcctacat cgacctgtac gatgtgaaga acggcaagat cgaccctctg     120

cagctgatca ccctgaactc tcccgatctg aaggcccagt acgtgatcag acaaggcggc     180

aactacttca cccagccttc cgagctgacc accgtgggag ccgcctctat caattacacc     240

gtgctgaaaa ccgacggctc ccctcacacc aagcctgatg gacaggtgga catcatcaac 300

gtgtccctga ccatctacaa cagcagcgcc ctgcgggaca agatcgatga agtgaagaag 360

aaggccgagg atcccaagtg ggacgagggc tctagagaca aggtgctgat ctccctggac 420

gacatcaaga ccgacatcga caacaacccc aagacacagt ccgatatcgc caacaagatc 480

accgaagtga ccaacctcga gaagatcctg gtgcctcgga tcccc 525

<210> 6
<211> 525
<212> RNA
<213> Streptococcus sp.

<400> 6
auggccgaag ugaucuccgg cucugcugug acccugaaca ccaacaugac caagaacgug 60

cagaacggca gagccuacau cgaccuguac gaugugaaga acggcaagau cgacccucug 120

cagcugauca cccugaacuc ucccgaucug aaggcccagu acgugaucag acaaggcggc 180

aacuacuuca cccagccuuc cgagcugacc accgugggag ccgccucuau caauuacacc 240

gugcugaaaa ccgacggcuc cccucacacc aagccugaug gacaggugga caucaucaac 300

gugucccuga ccaucuacaa cagcagcgcc cugcgggaca agaucgauga agugaagaag 360

aaggccgagg aucccaagug ggacgagggc ucuagagaca aggugcugau cucccuggac 420

gacaucaaga ccgacaucga caacaacccc aagacacagu ccgauaucgc caacaagauc 480

accgaaguga ccaaccucga gaagauccug gugccucgga ucccc 525

<210> 7
<211> 175
<212> PRT
<213> Streptococcus sp.

<400> 7

Met Ala Glu Val Ile Ser Gly Ser Ala Val Thr Leu Asn Thr Asn Met
1               5                   10                  15

Thr Lys Asn Val Gln Asn Gly Arg Ala Tyr Ile Asp Leu Tyr Asp Val
            20                  25                  30

Lys Asn Gly Lys Ile Asp Pro Leu Gln Leu Ile Thr Leu Asn Ser Pro
        35                  40                  45

Asp Leu Lys Ala Gln Tyr Val Ile Arg Gln Gly Gly Asn Tyr Phe Thr
    50                  55                  60

Gln Pro Ser Glu Leu Thr Thr Val Gly Ala Ala Ser Ile Asn Tyr Thr
65                  70                  75                  80

Val Leu Lys Thr Asp Gly Ser Pro His Thr Lys Pro Asp Gly Gln Val
                85                  90                  95

Asp Ile Ile Asn Val Ser Leu Thr Ile Tyr Asn Ser Ser Ala Leu Arg
            100                 105                 110

Asp Lys Ile Asp Glu Val Lys Lys Lys Ala Glu Asp Pro Lys Trp Asp
        115                 120                 125

Glu Gly Ser Arg Asp Lys Val Leu Ile Ser Leu Asp Asp Ile Lys Thr
    130                 135                 140

Asp Ile Asp Asn Asn Pro Lys Thr Gln Ser Asp Ile Ala Asn Lys Ile
145                 150                 155                 160

Thr Glu Val Thr Asn Leu Glu Lys Ile Leu Val Pro Arg Ile Pro
                165                 170                 175

<210>  8
<211>  516
<212>  DNA
<213>  Streptococcus sp.

<400>  8
tctacaattc cagggagtgc agcgacctta aatacaagca tcactaaaaa tatacaaaac      60

ggaaatgctt acatagattt atatgatgta aaattaggta aaatagatcc attacaatta     120

attgttttag aacaaggttt tacagcaaag tatgttttta gacaaggtac taaatactat     180

ggggatgttt ctcagttgca gagtacagga agggctagtc ttacctataa tatatttggt     240

gaagatggac taccacatgt aaagactgat ggacaaattg atatagttag tgttgcttta     300

actatttatg attcaacaac cttgagggat aagattgaag aagttagaac gaatgcaaac     360

gatcctaagt ggacggaaga aagtcgtact gaggttttaa caggattaga tacaattaag     420

acagatattg ataataatcc taagacgcaa acagatattg atagtaaaat tgttgaggtt     480

aatgaattag agaaattgtt agtattgtca taataa                               516

<210>  9
<211>  516
<212>  RNA
<213>  Streptococcus sp.

<400>  9
ucuacaauuc cagggagugc agcgaccuua aauacaagca ucacuaaaaa uauacaaaac      60

ggaaaugcuu acauagauuu auaugaugua aaauuaggua aaauagaucc auuacaauua     120

auuguuuuag aacaagguuu uacagcaaag uauguuuuua gacaagguac uaaauacuau     180

ggggauguuu cucaguugca gaguacagga agggcuaguc uuaccuauaa uauauuuggu     240

gaagauggac uaccacaugu aaagacugau ggacaaauug auauaguuag uguugcuuua 300

acuauuuaug auucaacaac cuugagggau aagauugaag aaguuagaac gaaugcaaac 360

gauccuaagu ggacggaaga aagucguacu gagguuuuaa caggauuaga uacaauuaag 420

acagauauug auaauaaucc uaagacgcaa acagauauug auaguaaaau uguugagguu 480

aaugaauuag agaaauuguu aguauuguca uaauaa 516

<210> 10
<211> 516
<212> DNA
<213> Streptococcus sp.

<400> 10
agcaccattc cgggtagcgc agcaacactg aatacaagca ttaccaaaaa cattcagaac 60

ggcaacgcat atatcgatct gtacgacgtt aaactgggta aaattgatcc tctgcaactg 120

atcgttctgg aacagggttt taccgcaaaa tatgtgtttc gtcagggcac caaatactat 180

ggtgatgtta gccagctgca gagcaccggt cgtgcaagcc tgacctataa catttttggt 240

gaagatggtc tgccgcatgt taaaaccgat ggccagattg atatcgttag cgttgcactg 300

acgatttatg atagcaccac cctgcgcgat aaaatcgaag aagttcgtac caacgcaaac 360

gatcctaaat ggaccgaaga aagccgtacc gaagttctga ccggtctgga taccattaaa 420

acggatatcg acaataatcc taaaacgcag acagatattg atagcaaaat tgtggaagtg 480

aatgaactgg aaaaactgct ggttctgagc taataa 516

<210> 11
<211> 516
<212> RNA
<213> Streptococcus sp.

<400> 11
agcaccauuc cggguagcgc agcaacacug aauacaagca uuaccaaaaa cauucagaac 60

ggcaacgcau auaucgaucu guacgacguu aaacugggua aaauugaucc ucugcaacug 120

aucguucugg aacaggguuu uaccgcaaaa uauguguuuc gucagggcac caaauacuau 180

ggugauguua gccagcugca gagcaccggu cgugcaagcc ugaccuauaa cauuuuuggu 240

gaagaugguc ugccgcaugu uaaaaccgau ggccagauug auaucguuag cguugcacug 300

acgauuuaug auagcaccac ccugcgcgau aaaaucgaag aaguucguac caacgcaaac 360

gauccuaaau ggaccgaaga aagccguacc gaaguucuga ccggucugga uaccauuaaa 420

acggauaucg acaauaaucc uaaaacgcag acagauauug auagcaaaau uguggaagug 480

aaugaacugg aaaaacugcu gguucugagc uaauaa 516

<210> 12
<211> 516

<212>  DNA
<213>  Streptococcus sp.

<400>  12
agcacaatcc ctggatctgc cgccacactg aacacctcta tcaccaagaa catccagaat      60

gggaacgctt acatcgatct ctacgacgtc aagctgggca agattgatcc cctccagctc     120

atcgtgctgg aacagggctt caccgccaaa tacgtgttcc ggcagggcac caagtactac     180

ggggatgtgt cacagctgca gtccaccggc agagcttccc tgacctacaa catcttcggc     240

gaggatggcc tgcctcacgt gaaaacagat ggccagatcg acatcgtgtc tgtggctctg     300

acaatctacg actccaccac actgcgcgat aagattgagg aagtgcggac caacgccaac     360

gatcctaagt ggaccgagga atccagaacc gaggtgctga ccggcctgga caccattaag     420

acagatattg ataacaatcc taagactcag acagacatcg attccaagat cgtggaagtc     480

aacgagctgg aaaagctgct ggtgctgtcc tgatga                               516


<210>  13
<211>  516
<212>  RNA
<213>  Streptococcus sp.

<400>  13
agcacaaucc cuggaucugc cgccacacug aacaccucua ucaccaagaa cauccagaau      60

gggaacgcuu acaucgaucu cuacgacguc aagcugggca agauugaucc ccuccagcuc     120

aucgugcugg aacagggcuu caccgccaaa uacguguucc ggcagggcac caaguacuac     180

ggggaugugu cacagcugca guccaccggc agagcuuccc ugaccuacaa caucuucggc     240

gaggauggcc ugccucacgu gaaaacagau ggccagaucg acaucguguc uguggcucug     300

acaaucuacg acuccaccac acugcgcgau aagauugagg aagugcggac caacgccaac     360

gauccuaagu ggaccgagga auccagaacc gaggugcuga ccggccugga caccauuaag     420

acagauauug auaacaaucc uaagacucag acagacaucg auuccaagau cguggaaguc     480

aacgagcugg aaaagcugcu ggugcugucc ugauga                               516


<210>  14
<211>  170
<212>  PRT
<213>  Streptococcus sp.

<400>  14

Ser Thr Ile Pro Gly Ser Ala Ala Thr Leu Asn Thr Ser Ile Thr Lys
1               5                   10                  15


Asn Ile Gln Asn Gly Asn Ala Tyr Ile Asp Leu Tyr Asp Val Lys Leu
            20                  25                  30

Gly Lys Ile Asp Pro Leu Gln Leu Ile Val Leu Glu Gln Gly Phe Thr
        35                  40                  45

Ala Lys Tyr Val Phe Arg Gln Gly Thr Lys Tyr Tyr Gly Asp Val Ser
    50                  55                  60

Gln Leu Gln Ser Thr Gly Arg Ala Ser Leu Thr Tyr Asn Ile Phe Gly
65                  70                  75                  80

Glu Asp Gly Leu Pro His Val Lys Thr Asp Gly Gln Ile Asp Ile Val
                85                  90                  95

Ser Val Ala Leu Thr Ile Tyr Asp Ser Thr Thr Leu Arg Asp Lys Ile
            100                 105                 110

Glu Glu Val Arg Thr Asn Ala Asn Asp Pro Lys Trp Thr Glu Glu Ser
        115                 120                 125

Arg Thr Glu Val Leu Thr Gly Leu Asp Thr Ile Lys Thr Asp Ile Asp
    130                 135                 140

Asn Asn Pro Lys Thr Gln Thr Asp Ile Asp Ser Lys Ile Val Glu Val
145                 150                 155                 160

Asn Glu Leu Glu Lys Leu Leu Val Leu Ser
                165                 170

<210>  15
<211>  1047
<212>  DNA
<213>  Streptococcus sp.

<400>  15
atggctgaag taatttcagg aagtgctgtt acgttaaaca caaatatgac taaaaatgtt      60

cagaatggta gagcatatat agatttatat gatgtgaaaa atgggaaaat agatccatta     120

caattaatta cgttaaattc acctgattta aaagctcagt atgtcattag gcaaggcggc     180

aattatttca cacaaccttc tgaattgact actgttggtg cagctagtat taattataca     240

gtattgaaga cagatggaag tcctcatacg aagcctgatg gacaagtgga tattataaac     300

gtttcattga ctatttacaa ttcttcagct ttgagagata aaatagatga agttaaaaag     360

aaagcggaag accctaaatg ggacgaggga agtcgcgata aagttttgat aagtttagat     420

gatatcaaaa cagatattga taataatcct aagacgcaat cagacattgc caataaaata     480

actgaagtta ctaatttaga aaaaatacta gtacctcgaa tcccagaatt ctctacaatt     540

ccagggagtg cagcgacctt aaatacaagc atcactaaaa atatacaaaa cggaaatgct     600

tacatagatt tatatgatgt aaaattaggt aaaatagatc cattacaatt aattgtttta     660

gaacaaggtt ttacagcaaa gtatgttttt agacaaggta ctaaatacta tggggatgtt 720

tctcagttgc agagtacagg aagggctagt cttacctata atatatttgg tgaagatgga 780

ctaccacatg taaagactga tggacaaatt gatatagtta gtgttgcttt aactatttat 840

gattcaacaa ccttgaggga taagattgaa gaagttagaa cgaatgcaaa cgatcctaag 900

tggacggaag aaagtcgtac tgaggtttta acaggattag atacaattaa gacagatatt 960

gataataatc ctaagacgca aacagatatt gatagtaaaa ttgttgaggt taatgaatta 1020

gagaaattgt tagtattgtc ataataa 1047

<210> 16
<211> 1047
<212> RNA
<213> Streptococcus sp.

<400> 16
auggcugaag uaauuucagg aagugcuguu acguuaaaca caaauaugac uaaaaauguu 60

cagaauggua gagcauauau agauuuauau gaugugaaaa augggaaaau agauccauua 120

caauuaauua cguuaaauuc accugauuua aaagcucagu augucauuag gcaaggcggc 180

aauuauuuca cacaaccuuc ugaauugacu acuguuggug cagcuaguau uaauuauaca 240

guauugaaga cagauggaag uccucauacg aagccugaug gacaagugga uauuauaaac 300

guuucauuga cuauuuacaa uucuucagcu uugagagaua aaauagauga aguuaaaaag 360

aaagcggaag acccuaaaug ggacgaggga agucgcgaua aaguuuugau aaguuuagau 420

gauaucaaaa cagauauuga uaauaauccu aagacgcaau cagacauugc caauaaaaua 480

acugaaguua cuaauuuaga aaaaauacua guaccucgaa ucccagaauu cucuacaauu 540

ccagggagug cagcgaccuu aaauacaagc aucacuaaaa auauacaaaa cggaaaugcu 600

uacauagauu uauaugaugu aaaauuaggu aaaauagauc cauuacaauu aauuguuuua 660

gaacaagguu uuacagcaaa guauguuuuu agacaaggua cuaaauacua uggggauguu 720

ucucaguugc agaguacagg aagggcuagu cuuaccuaua auauauuugg ugaagaugga 780

cuaccacaug uaaagacuga uggacaaauu gauauaguua guguugcuuu aacuauuuau 840

gauucaacaa ccuugaggga uaagauugaa gaaguuagaa cgaaugcaaa cgauccuaag 900

uggacggaag aaagucguac ugagguuuua acaggauuag auacaauuaa gacagauauu 960

gauaauaauc cuaagacgca aacagauauu gauaguaaaa uuguugaggu uaaugaauua 1020

gagaaauugu uaguauuguc auaauaa 1047

<210> 17
<211> 1047
<212> DNA
<213> Streptococcus sp.

<400>  17
atggccgaag ttattagcgg tagcgcagtt accctgaata ccaatatgac caaaaatgtg      60

cagaatggcc gtgcctatat tgatctgtat gatgtgaaaa atggcaaaat cgatccgctg     120

cagctgatta ccctgaattc tccggatctg aaagcacagt atgttattcg tcagggtggt     180

aactatttta cccagccgag cgaactgacc accgttggtg cagcaagcat taactatacc     240

gttctgaaaa ccgatggtag tccgcatacc aaaccggatg gtcaggttga tattatcaat     300

gttagcctga ccatctataa tagcagcgca ctgcgtgata aaattgatga agtgaaaaag     360

aaagccgaag atccgaaatg ggatgaaggt agccgtgata aagttctgat tagcctggat     420

gatatcaaaa ccgatattga taacaacccg aaaacccaga gcgatattgc aaacaaaatt     480

accgaagtga ccaatctgga aaaaattctg gttccgcgta ttccggaatt tagcaccatt     540

ccgggtagcg cagcaacact gaatacaagc attaccaaaa acattcagaa cggcaacgca     600

tatatcgatc tgtacgacgt taaactgggt aaaattgatc ctctgcaact gatcgttctg     660

gaacagggtt ttaccgcaaa atatgtgttt cgtcagggca ccaaatacta tggtgatgtt     720

agccagctgc agagcaccgg tcgtgcaagc ctgacctata acattttggt tgaagatggt     780

ctgccgcatg ttaaaaccga tggccagatt gatatcgtta gcgttgcact gacgatttat     840

gatagcacca ccctgcgcga taaaatcgaa gaagttcgta ccaacgcaaa cgatcctaaa     900

tggaccgaag aaagccgtac cgaagttctg accggtctgg ataccattaa aacggatatc     960

gacaataatc ctaaaacgca gacagatatt gatagcaaaa ttgtggaagt gaatgaactg    1020

gaaaaactgc tggttctgag ctaataa                                        1047

<210>  18
<211>  1047
<212>  RNA
<213>  Streptococcus sp.

<400>  18
auggccgaag uuauuagcgg uagcgcaguu acccugaaua ccaauaugac caaaaaugug      60

cagaauggcc gugccuauau ugaucuguau gaugugaaaa auggcaaaau cgauccgcug     120

cagcugauua cccugaauuc uccggaucug aaagcacagu auguuauucg ucaggguggu     180

aacuauuuua cccagccgag cgaacugacc accguuggug cagcaagcau uaacuauacc     240

guucugaaaa ccgaugguag uccgcauacc aaaccggaug gucagguuga uauuaucaau     300

guuagccuga ccaucuauaa uagcagcgca cugcgugaua aaauugauga agugaaaaag     360

aaagccgaag auccgaaaug ggaugaaggu agccgugaua aaguucugau uagccuggau     420

gauaucaaaa ccgauauuga uaacaacccg aaaacccaga gcgauauugc aaacaaaauu     480

accgaaguga ccaaucugga aaaaauucug guuccgcgua uuccggaauu uagcaccauu     540

ccggguagcg cagcaacacu gaauacaagc auuaccaaaa acauucagaa cggcaacgca 600

uauaucgauc uguacgacgu uaaacugggu aaaauugauc cucugcaacu gaucguucug 660

gaacaggguu uuaccgcaaa auauguguuu cgucagggca ccaaauacua uggugauguu 720

agccagcugc agagcaccgg ucgugcaagc cugaccuaua acauuuuugg ugaagauggu 780

cugccgcaug uuaaaaccga uggccagauu gauaucguua gcguugcacu gacgauuuau 840

gauagcacca cccugcgcga uaaaaucgaa gaaguucgua ccaacgcaaa cgauccuaaa 900

uggaccgaag aaagccguac cgaaguucug accggucugg auaccauuaa aacggauauc 960

gacaauaauc cuaaaacgca gacagauauu gauagcaaaa uuguggaagu gaaugaacug 1020

gaaaaacugc ugguucugag cuaauaa 1047

<210> 19
<211> 1047
<212> DNA
<213> Streptococcus sp.

<400> 19
atggccgaag tgatctccgg ctctgctgtg accctgaaca ccaacatgac caagaacgtg 60

cagaacggca gagcctacat cgacctgtac gatgtgaaga acggcaagat cgaccctctg 120

cagctgatca ccctgaactc tcccgatctg aaggcccagt acgtgatcag acaaggcggc 180

aactacttca cccagccttc cgagctgacc accgtgggag ccgcctctat caattacacc 240

gtgctgaaaa ccgacggctc ccctcacacc aagcctgatg gacaggtgga catcatcaac 300

gtgtccctga ccatctacaa cagcagcgcc ctgcgggaca agatcgatga agtgaagaag 360

aaggccgagg atcccaagtg ggacgagggc tctagagaca aggtgctgat ctccctggac 420

gacatcaaga ccgacatcga caacaacccc aagacacagt ccgatatcgc caacaagatc 480

accgaagtga ccaacctcga gaagatcctg gtgcctcgga tccccgagtt cagcacaatc 540

cctggatctg ccgccacact gaacacctct atcaccaaga acatccagaa tgggaacgct 600

tacatcgatc tctacgacgt caagctgggc aagattgatc ccctccagct catcgtgctg 660

gaacagggct tcaccgccaa atacgtgttc cggcagggca ccaagtacta cggggatgtg 720

tcacagctgc agtccaccgg cagagcttcc ctgacctaca acatcttcgg cgaggatggc 780

ctgcctcacg tgaaaacaga tggccagatc gacatcgtgt ctgtggctct gacaatctac 840

gactccacca cactgcgcga taagattgag gaagtgcgga ccaacgccaa cgatcctaag 900

tggaccgagg aatccagaac cgaggtgctg accggcctgg acaccattaa gacagatatt 960

gataacaatc ctaagactca gacagacatc gattccaaga tcgtggaagt caacgagctg 1020

gaaaagctgc tggtgctgtc ctgatga 1047

<210> 20

<211>  1047
<212>  RNA
<213>  Streptococcus sp.

<400>  20
auggccgaag ugaucuccgg cucugcugug acccugaaca ccaacaugac caagaacgug      60

cagaacggca gagccuacau cgaccuguac gaugugaaga acggcaagau cgacccucug     120

cagcugauca cccugaacuc ucccgaucug aaggcccagu acgugaucag acaaggcggc     180

aacuacuuca cccagccuuc cgagcugacc accgugggag ccgccucuau caauuacacc     240

gugcugaaaa ccgacggcuc cccucacacc aagccugaug gacaggugga caucaucaac     300

gugucccuga ccaucuacaa cagcagcgcc cugcgggaca agaucgauga agugaagaag     360

aaggccgagg aucccaagug ggacgagggc ucuagagaca aggugcugau cucccuggac     420

gacaucaaga ccgacaucga caacaacccc aagacacagu ccgauaucgc caacaagauc     480

accgaaguga ccaaccucga gaagauccug gugccucgga uccccgaguu cagcacaauc     540

ccuggaucug ccgccacacu gaacaccucu aucaccaaga acauccagaa ugggaacgcu     600

uacaucgauc ucuacgacgu caagcugggc aagauugauc cccuccagcu caucgugcug     660

gaacagggcu ucaccgccaa auacguguuc cggcagggca ccaaguacua cggggaugug     720

ucacagcugc aguccaccgg cagagcuucc cugaccuaca acaucuucgg cgaggauggc     780

cugccucacg ugaaaacaga uggccagauc gacaucgugu cuguggcucu gacaaucuac     840

gacuccacca cacugcgcga uaagauugag gaagugcgga ccaacgccaa cgauccuaag     900

uggaccgagg aauccagaac cgaggugcug accggccugg acaccauuaa gacagauauu     960

gauaacaauc cuaagacuca gacagacauc gauuccaaga ucguggaagu caacgagcug    1020

gaaaagcugc uggugcuguc cugauga                                        1047


<210>  21
<211>  347
<212>  PRT
<213>  Streptococcus sp.

<400>  21

Met Ala Glu Val Ile Ser Gly Ser Ala Val Thr Leu Asn Thr Asn Met
1               5                   10                  15


Thr Lys Asn Val Gln Asn Gly Arg Ala Tyr Ile Asp Leu Tyr Asp Val
            20                  25                  30


Lys Asn Gly Lys Ile Asp Pro Leu Gln Leu Ile Thr Leu Asn Ser Pro
        35                  40                  45


Asp Leu Lys Ala Gln Tyr Val Ile Arg Gln Gly Gly Asn Tyr Phe Thr
    50                  55                  60

Gln Pro Ser Glu Leu Thr Thr Val Gly Ala Ala Ser Ile Asn Tyr Thr
65                  70                  75                  80

Val Leu Lys Thr Asp Gly Ser Pro His Thr Lys Pro Asp Gly Gln Val
                85                  90                  95

Asp Ile Ile Asn Val Ser Leu Thr Ile Tyr Asn Ser Ser Ala Leu Arg
            100                 105                 110

Asp Lys Ile Asp Glu Val Lys Lys Lys Ala Glu Asp Pro Lys Trp Asp
        115                 120                 125

Glu Gly Ser Arg Asp Lys Val Leu Ile Ser Leu Asp Asp Ile Lys Thr
    130                 135                 140

Asp Ile Asp Asn Asn Pro Lys Thr Gln Ser Asp Ile Ala Asn Lys Ile
145                 150                 155                 160

Thr Glu Val Thr Asn Leu Glu Lys Ile Leu Val Pro Arg Ile Pro Glu
                165                 170                 175

Phe Ser Thr Ile Pro Gly Ser Ala Ala Thr Leu Asn Thr Ser Ile Thr
            180                 185                 190

Lys Asn Ile Gln Asn Gly Asn Ala Tyr Ile Asp Leu Tyr Asp Val Lys
        195                 200                 205

Leu Gly Lys Ile Asp Pro Leu Gln Leu Ile Val Leu Glu Gln Gly Phe
    210                 215                 220

Thr Ala Lys Tyr Val Phe Arg Gln Gly Thr Lys Tyr Tyr Gly Asp Val
225                 230                 235                 240

Ser Gln Leu Gln Ser Thr Gly Arg Ala Ser Leu Thr Tyr Asn Ile Phe
                245                 250                 255

Gly Glu Asp Gly Leu Pro His Val Lys Thr Asp Gly Gln Ile Asp Ile
            260                 265                 270

Val Ser Val Ala Leu Thr Ile Tyr Asp Ser Thr Thr Leu Arg Asp Lys
        275                 280                 285

Ile Glu Glu Val Arg Thr Asn Ala Asn Asp Pro Lys Trp Thr Glu Glu
    290                 295                 300

Ser Arg Thr Glu Val Leu Thr Gly Leu Asp Thr Ile Lys Thr Asp Ile

305                 310                 315                 320

Asp Asn Asn Pro Lys Thr Gln Thr Asp Ile Asp Ser Lys Ile Val Glu
                325                 330                 335

Val Asn Glu Leu Glu Lys Leu Leu Val Leu Ser
            340                 345

<210>  22
<211>  519
<212>  DNA
<213>  Streptococcus sp.

<400>  22
atggccgaag ttattagcgg tagcgcagca accctgaata gcgcactggt taaaaatgtt      60

agcggtggca aagcctatat cgacatctat gatgtgaaaa acggcaaaat tgatccgctg     120

aatctgattg ttctgcctcc gagcaattat agcgccaact attatatcaa acagggtggt     180

cgcattttca ccaatgttaa tcagctgcag acaccgggta cagcaaccat tacctataac     240

attctggatg aaaatggcaa cccgtatacc aaaagtgatg gccagattga tattgttagc     300

ctggttacca ccgtttatga taccaccgaa ctgcgcaata acatcaacaa agttattgag     360

aatgccaacg acccgaaatg gtcagatgat agccgtaaag atgttctgag caaaatcgag     420

gtgatcaaaa acgatattga taacaacccg aaaacccaga gcgatatcga caacaaaatt     480

gtggaagtga acgagctgga aaaactgctg gttctgccg                            519

<210>  23
<211>  519
<212>  RNA
<213>  Streptococcus sp.

<400>  23
auggccgaag uuauuagcgg uagcgcagca acccugaaua gcgcacuggu uaaaaauguu      60

agcgguggca aagccuauau cgacaucuau gaugugaaaa acggcaaaau ugauccgcug     120

aaucugauug uucugccucc gagcaauuau agcgccaacu auuauaucaa acaggguggu    180

cgcauuuuca ccaauguuaa ucagcugcag acaccgggua cagcaaccau uaccuauaac     240

auucuggaug aaaauggcaa cccguauacc aaaagugaug gccagauuga uauuguuagc     300

cugguuacca ccguuuauga uaccaccgaa cugcgcaaua acaucaacaa aguuauugag     360

aaugccaacg acccgaaaug gucagaugau agccguaaag auguucugag caaaaucgag     420

gugaucaaaa acgauauuga uaacaacccg aaaacccaga gcgauaucga caacaaaauu     480

guggaaguga acgagcugga aaaacugcug guucugccg                            519

<210>  24
<211>  519

<212>  DNA
<213>  Streptococcus sp.

<400>  24
atggccgaag ttattagcgg tagcgcagca accctgaata gcgcactggt taaaaatgtt      60

agcggtggca aagcctatat cgacatctat gatgtgaaaa acggcaaaat tgatccgctg     120

aatctgattg ttctgacccc gagcaattat agcgccaact attatatcaa acagggtggt     180

cgtattttca ccagcgttaa tcagctgcag acaccgggta cagcaaccat tacctataac     240

attctggatg aaaatggcaa cccgtatacc aaaagtgatg gccagattga tattgttagc     300

ctggttacca ccgtttatga taccaccgaa ctgcgcaata acatcaacaa agttattgag     360

aatgccaacg acccgaaatg gtcagatgat agccgtaaag atgttctgag caaaatcgag     420

gtgatcaaaa acgatattga taacaacccg aaaacccaga gcgatatcga caacaaaatt     480

gtggaagtga acgagctgga aaaactgctg gttctgccg                            519


<210>  25
<211>  519
<212>  RNA
<213>  Streptococcus sp.

<400>  25
auggccgaag uuauuagcgg uagcgcagca acccugaaua gcgcacuggu uaaaaauguu      60

agcgguggca aagccuauau cgacaucuau gaugugaaaa acggcaaaau ugauccgcug     120

aaucugauug uucugacccc gagcaauuau agcgccaacu auuauaucaa acaggguggu     180

cguauuuuca ccagcguuaa ucagcugcag acaccgggua cagcaaccau uaccuauaac     240

auucuggaug aaaauggcaa cccguauacc aaaagugaug gccagauuga uauuguuagc     300

cugguuacca ccguuuauga uaccaccgaa cugcgcaaua acaucaacaa aguuauugag     360

aaugccaacg acccgaaaug gucagaugau agccguaaag auguucugag caaaaucgag     420

gugaucaaaa acgauauuga uaacaacccg aaaacccaga gcgauaucga caacaaaauu     480

guggaaguga acgagcugga aaaacugcug guucugccg                            519


<210>  26
<211>  519
<212>  DNA
<213>  Streptococcus sp.

<400>  26
atggccgaag tgatctccgg ctctgctgct accctgaact ctgccctggt caagaatgtg      60

tctggcggca aggcctacat cgacatctac gacgtgaaga acggcaagat cgaccctctg     120

aacctgatcg tgctgacccc ttccaactac tccgccaact actacatcaa gcaaggcggc     180

agaatcttca cctccgtgaa ccagctgcag acccctggca ccgctaccat cacctacaac     240

atcctggacg agaatggcaa cccctacacc aagtccgacg gccagatcga tatcgtgtcc     300

ctggtcacca ccgtgtacga caccaccgag ctgcggaaca acatcaacaa agtgatcgag 360

aacgccaacg atcccaagtg gagcgacgac tctagaaagg acgtgctgtc caagatcgaa 420

gtcatcaaga acgacatcga caacaacccc aagacacagt ccgacattga taacaagatc 480

gtggaagtga acgagctgga aaagctgctg gtgctgccc 519

<210> 27
<211> 519
<212> RNA
<213> Streptococcus sp.

<400> 27
auggccgaag ugaucuccgg cucugcugcu acccugaacu cugcccuggu caagaaugug 60

ucuggcggca aggccuacau cgacaucuac gacgugaaga acggcaagau cgacccucug 120

aaccugaucg ugcugacccc uuccaacuac uccgccaacu acuacaucaa gcaaggcggc 180

agaaucuuca ccuccgugaa ccagcugcag accccuggca ccgcuaccau caccuacaac 240

auccuggacg agaauggcaa ccccuacacc aaguccgacg gccagaucga uaucgugucc 300

cuggucacca ccguguacga caccaccgag cugcggaaca acaucaacaa agugaucgag 360

aacgccaacg aucccaagug gagcgacgac ucuagaaagg acgugcuguc caagaucgaa 420

gucaucaaga acgacaucga caacaacccc aagacacagu ccgacauuga uaacaagauc 480

guggaaguga acgagcugga aaagcugcug gugcugccc 519

<210> 28
<211> 173
<212> PRT
<213> Streptococcus sp.

<400> 28

Met Ala Glu Val Ile Ser Gly Ser Ala Ala Thr Leu Asn Ser Ala Leu
1               5                   10                  15

Val Lys Asn Val Ser Gly Gly Lys Ala Tyr Ile Asp Ile Tyr Asp Val
            20                  25                  30

Lys Asn Gly Lys Ile Asp Pro Leu Asn Leu Ile Val Leu Thr Pro Ser
        35                  40                  45

Asn Tyr Ser Ala Asn Tyr Tyr Ile Lys Gln Gly Gly Arg Ile Phe Thr
    50                  55                  60

Ser Val Asn Gln Leu Gln Thr Pro Gly Thr Ala Thr Ile Thr Tyr Asn
65                  70                  75                  80

Ile Leu Asp Glu Asn Gly Asn Pro Tyr Thr Lys Ser Asp Gly Gln Ile

85                  90                  95

Asp Ile Val Ser Leu Val Thr Thr Val Tyr Asp Thr Thr Glu Leu Arg
            100                 105                 110

Asn Asn Ile Asn Lys Val Ile Glu Asn Ala Asn Asp Pro Lys Trp Ser
        115                 120                 125

Asp Asp Ser Arg Lys Asp Val Leu Ser Lys Ile Glu Val Ile Lys Asn
    130                 135                 140

Asp Ile Asp Asn Asn Pro Lys Thr Gln Ser Asp Ile Asp Asn Lys Ile
145                 150                 155                 160

Val Glu Val Asn Glu Leu Glu Lys Leu Leu Val Leu Pro
                165                 170

<210>  29
<211>  522
<212>  DNA
<213>  Streptococcus sp.

<400>  29
agcaccattc cgggttcagc agccacactg aataccagca ttaccaaaaa cattcagaat      60

ggcaacgcct acattgatct gtacgatgta aaaaatggtc tgatcgatcc gcagaacctg     120

atcgtgctga atccgagcag ctattcagcc aattattata ttaaacaagg cgcaaaatac     180

tatagcaacc cgagcgaaat taccaccacc ggtagcgcca ccattacgtt taatatcctg     240

gacgaaaccg gtaacccgca taaaaaagca gatggtcaaa ttgatatcgt gagcgttaac     300

ctgacgattt atgatagcac agccctgcgt aatcgtattg atgaagtgat taataacgcg     360

aatgatccta aatggtccga tggtagtcgt gatgaagtac tgaccggtct ggaaaaaatc     420

aaaaaagaca tcgacaataa tccgaaaacg cagattgaca ttgacaataa aatcaacgag     480

gtgaacgaga tcgagaaact gctggtagtt agcctgtaat aa                        522

<210>  30
<211>  522
<212>  RNA
<213>  Streptococcus sp.

<400>  30
agcaccauuc cggguucagc agccacacug aauaccagca uuaccaaaaa cauucagaau      60

ggcaacgccu acauugaucu guacgaugua aaaaaugguc ugaucgaucc gcagaaccug     120

aucgugcuga auccgagcag cuauucagcc aauuauuaua uuaaacaagg cgcaaaauac     180

uauagcaacc cgagcgaaau uaccaccacc gguagcgcca ccauuacguu uaauauccug     240

gacgaaaccg guaacccgca uaaaaaagca gauggucaaa uugauaucgu gagcguuaac     300

cugacgauuu augauagcac agcccugcgu aaucguauug augaagugau uaauaacgcg 360

aaugauccua aaugguccga ugguagucgu gaugaaguac ugaccggucu ggaaaaaauc 420

aaaaaagaca ucgacaauaa uccgaaaacg cagauugaca uugacaauaa aaucaacgag 480

gugaacgaga ucgagaaacu gcugguaguu agccuguaau aa 522

<210> 31
<211> 522
<212> DNA
<213> Streptococcus sp.

<400> 31
agcaccattc cgggttcagc agccacactg aataccagca ttaccaaaaa cattcagaat 60

ggcaacgcct acattgatct gtacgatgta aaaaatggtc tgatcgatcc gcagaacctg 120

atcgtgctga atccgagcag ctattcagcc aattattata ttaaacaagg cgcaaaatac 180

tatagcaacc cgagcgaaat taccaccacc ggtagcgcca ccattacgtt taatatcctg 240

gacgaaaccg gtaacccgca taaaaaagca gatggtcaaa ttgatatcgt gagcgttaac 300

ctgacgattt atgatagcac agccctgcgt aatcgtattg atgaagtgat taataacgcg 360

aatgatccta aatggtccga tggtagtcgt gatgaagtac tgaccggtct ggaaaaaatc 420

aaaaaagaca tcgacaataa tccgaaaacg cagattgaca ttgacaataa aatcaacgag 480

gtgaacgaga tcgagaaact gctggtagtt agcctgtaat aa 522

<210> 32
<211> 522
<212> RNA
<213> Streptococcus sp.

<400> 32
agcaccauuc cggguucagc agccacacug aauaccagca uuaccaaaaa cauucagaau 60

ggcaacgccu acauugaucu guacgaugua aaaaaugguc ugaucgaucc gcagaaccug 120

aucgugcuga auccgagcag cuauucagcc aauuauuaua uuaaacaagg cgcaaaauac 180

uauagcaacc cgagcgaaau uaccaccacc gguagcgcca ccauuacguu uaauauccug 240

gacgaaaccg guaacccgca uaaaaaagca gauggucaaa uugauaucgu gagcguuaac 300

cugacgauuu augauagcac agcccugcgu aaucguauug augaagugau uaauaacgcg 360

aaugauccua aaugguccga ugguagucgu gaugaaguac ugaccggucu ggaaaaaauc 420

aaaaaagaca ucgacaauaa uccgaaaacg cagauugaca uugacaauaa aaucaacgag 480

gugaacgaga ucgagaaacu gcugguaguu agccuguaau aa 522

<210> 33
<211> 522
<212> DNA

<213>  Streptococcus sp.

<400>  33
agcacaatcc ctggatctgc cgccacactg aacacctcca tcaccaagaa tatccagaac      60

gggaacgctt acatcgacct gtatgatgtc aagaacgggc tgatcgatcc ccagaatctg     120

attgtgctca acccctcctc ctactctgct aattactata ttaagcaggg cgccaagtac     180

tactctaacc cctccgagat caccaccacc ggctccgcca ccatcacatt caatattctg     240

gatgagacag gcaaccctca caagaaggct gacggacaga ttgacattgt gtctgtgaac     300

ctgaccatct acgattccac cgctctgcgg aaccggatcg atgaagtgat caacaacgct     360

aatgacccta agtggagtga cggctccaga gatgaggtgc tgaccggcct ggaaaagatc     420

aagaaggata tcgataacaa tcctaaaact cagatcgata ttgacaacaa gatcaacgaa     480

gtcaacgaga tcgagaagct cctggtcgtg tctctgtgat ga                        522


<210>  34
<211>  522
<212>  RNA
<213>  Streptococcus sp.

<400>  34
agcacaaucc cuggaucugc cgccacacug aacaccucca ucaccaagaa uauccagaac      60

gggaacgcuu acaucgaccu guaugauguc aagaacgggc ugaucgaucc ccagaaucug     120

auugugcuca accccuccuc cuacucugcu aauuacuaua uuaagcaggg cgccaaguac     180

uacucuaacc ccuccgagau caccaccacc ggcuccgcca ccaucacauu caauauucug     240

gaugagacag gcaacccuca caagaaggcu gacggacaga uugacauugu gucugugaac     300

cugaccaucu acgauuccac cgcucugcgg aaccggaucg augaagugau caacaacgcu     360

aaugacccua aguggaguga cggcuccaga gaugaggugc ugaccggccu ggaaaagauc     420

aagaaggaua ucgauaacaa uccuaaaacu cagaucgaua uugacaacaa gaucaacgaa     480

gucaacgaga ucgagaagcu ccuggucgug ucucugugau ga                        522


<210>  35
<211>  172
<212>  PRT
<213>  Streptococcus sp.

<400>  35

Ser Thr Ile Pro Gly Ser Ala Ala Thr Leu Asn Thr Ser Ile Thr Lys
1               5                   10                  15


Asn Ile Gln Asn Gly Asn Ala Tyr Ile Asp Leu Tyr Asp Val Lys Asn
            20                  25                  30


Gly Leu Ile Asp Pro Gln Asn Leu Ile Val Leu Asn Pro Ser Ser Tyr

35                  40                  45

Ser Ala Asn Tyr Tyr Ile Lys Gln Gly Ala Lys Tyr Tyr Ser Asn Pro
    50                  55                  60

Ser Glu Ile Thr Thr Thr Gly Ser Ala Thr Ile Thr Phe Asn Ile Leu
65                  70                  75                  80

Asp Glu Thr Gly Asn Pro His Lys Lys Ala Asp Gly Gln Ile Asp Ile
                85                  90                  95

Val Ser Val Asn Leu Thr Ile Tyr Asp Ser Thr Ala Leu Arg Asn Arg
            100                 105                 110

Ile Asp Glu Val Ile Asn Asn Ala Asn Asp Pro Lys Trp Ser Asp Gly
        115                 120                 125

Ser Arg Asp Glu Val Leu Thr Gly Leu Glu Lys Ile Lys Lys Asp Ile
    130                 135                 140

Asp Asn Asn Pro Lys Thr Gln Ile Asp Ile Asp Asn Lys Ile Asn Glu
145                 150                 155                 160

Val Asn Glu Ile Glu Lys Leu Leu Val Val Ser Leu
                165                 170

<210>  36
<211>  1047
<212>  DNA
<213>  Streptococcus sp.

<400>  36
atggccgaag ttattagcgg tagcgcagca accctgaata gcgcactggt taaaaatgtt      60

agcggtggca aagcctatat cgacatctat gatgtgaaaa acggcaaaat tgatccgctg     120

aatctgattg ttctgcctcc gagcaattat agcgccaact attatatcaa acagggtggt     180

cgcattttca ccaatgttaa tcagctgcag acaccgggta cagcaaccat tacctataac     240

attctggatg aaaatggcaa cccgtatacc aaaagtgatg gccagattga tattgttagc     300

ctggttacca ccgtttatga taccaccgaa ctgcgcaata acatcaacaa agttattgag     360

aatgccaacg acccgaaatg gtcagatgat agccgtaaag atgttctgag caaaatcgag     420

gtgatcaaaa acgatattga taacaacccg aaaacccaga gcgatatcga caacaaaatt     480

gtggaagtga acgagctgga aaaactgctg gttctgccgg aatttagcac cattccgggt     540

tcagcagcca cactgaatac cagcattacc aaaaacattc agaatggcaa cgcctacatt     600

gatctgtacg atgtaaaaaa tggtctgatc gatccgcaga acctgatcgt gctgaatccg     660

agcagctatt cagccaatta ttatattaaa caaggcgcaa aatactatag caacccgagc 720

gaaattacca ccaccggtag cgccaccatt acgtttaata tcctggacga aaccggtaac 780

ccgcataaaa aagcagatgg tcaaattgat atcgtgagcg ttaacctgac gatttatgat 840

agcacagccc tgcgtaatcg tattgatgaa gtgattaata acgcgaatga tcctaaatgg 900

tccgatggta gtcgtgatga agtactgacc ggtctggaaa aaatcaaaaa agacatcgac 960

aataatccga aaacgcagat tgacattgac aataaaatca acgaggtgaa cgagatcgag 1020

aaactgctgg tagttagcct gtaataa 1047

<210> 37
<211> 1047
<212> RNA
<213> Streptococcus sp.

<400> 37
auggccgaag uuauuagcgg uagcgcagca acccugaaua gcgcacuggu uaaaaauguu 60

agcgguggca aagccuauau cgacaucuau gaugugaaaa acggcaaaau ugauccgcug 120

aaucugauug uucugccucc gagcaauuau agcgccaacu auuauaucaa acaggguggu 180

cgcauuuuca ccaauguuaa ucagcugcag acaccgggua cagcaaccau uaccuauaac 240

auucuggaug aaaauggcaa cccguauacc aaaagugaug gccagauuga uauuguuagc 300

cugguuacca ccguuuauga uaccaccgaa cugcgcaaua acaucaacaa aguuauugag 360

aaugccaacg acccgaaaug gucagaugau agccguaaag auguucugag caaaaucgag 420

gugaucaaaa acgauauuga uaacaacccg aaaacccaga gcgauaucga caacaaaauu 480

guggaaguga acgagcugga aaaacugcug guucugccgg aauuuagcac cauuccgggu 540

ucagcagcca cacugaauac cagcauuacc aaaaacauuc agaauggcaa cgccuacauu 600

gaucuguacg auguaaaaaa uggucugauc gauccgcaga accugaucgu gcugaauccg 660

agcagcuauu cagccaauua uuauauuaaa caaggcgcaa aauacuauag caacccgagc 720

gaaauuacca ccaccgguag cgccaccauu acguuuaaua uccuggacga aaccgguaac 780

ccgcauaaaa aagcagaugg ucaaauugau aucgugagcg uuaaccugac gauuuaugau 840

agcacagccc ugcguaaucg uauugaugaa gugauuaaua acgcgaauga uccuaaaugg 900

uccgauggua gucgugauga aguacugacc ggucuggaaa aaaucaaaaa agacaucgac 960

aauaauccga aaacgcagau ugacauugac aauaaaauca acgaggugaa cgagaucgag 1020

aaacugcugg uaguuagccu guaauaa 1047

<210> 38
<211> 1047
<212> DNA
<213> Streptococcus sp.

<400>  38
atggccgaag ttattagcgg tagcgcagca accctgaata gcgcactggt taaaaatgtt      60

agcggtggca aagcctatat cgacatctat gatgtgaaaa acggcaaaat tgatccgctg     120

aatctgattg ttctgacccc gagcaattat agcgccaact attatatcaa acagggtggt     180

cgtattttca ccagcgttaa tcagctgcag acaccgggta cagcaaccat tacctataac     240

attctggatg aaaatggcaa cccgtatacc aaaagtgatg gccagattga tattgttagc     300

ctggttacca ccgtttatga taccaccgaa ctgcgcaata acatcaacaa agttattgag     360

aatgccaacg acccgaaatg gtcagatgat agccgtaaag atgttctgag caaaatcgag     420

gtgatcaaaa acgatattga taacaacccg aaaacccaga gcgatatcga caacaaaatt     480

gtggaagtga acgagctgga aaaactgctg gttctgccgg aatttagcac cattccgggt     540

tcagcagcca cactgaatac cagcattacc aaaaacattc agaatggcaa cgcctacatt     600

gatctgtacg atgtaaaaaa tggtctgatc gatccgcaga acctgatcgt gctgaatccg     660

agcagctatt cagccaatta ttatattaaa caaggcgcaa aatactatag caacccgagc     720

gaaattacca ccaccggtag cgccaccatt acgtttaata tcctggacga aaccggtaac     780

ccgcataaaa aagcagatgg tcaaattgat atcgtgagcg ttaacctgac gatttatgat     840

agcacagccc tgcgtaatcg tattgatgaa gtgattaata acgcgaatga tcctaaatgg     900

tccgatggta gtcgtgatga agtactgacc ggtctggaaa aaatcaaaaa agacatcgac     960

aataatccga aaacgcagat tgacattgac aataaaatca acgaggtgaa cgagatcgag    1020

aaactgctgg tagttagcct gtaataa                                        1047

<210>  39
<211>  1047
<212>  RNA
<213>  Streptococcus sp.

<400>  39
auggccgaag uuauuagcgg uagcgcagca acccugaaua gcgcacuggu uaaaaauguu      60

agcgguggca aagccuauau cgacaucuau gaugugaaaa acggcaaaau ugauccgcug     120

aaucugauug uucugacccc gagcaauuau agcgccaacu auuauaucaa acaggguggu     180

cguauuuuca ccagcguuaa ucagcugcag acaccgggua cagcaaccau uaccuauaac     240

auucuggaug aaaauggcaa cccguauacc aaaagugaug gccagauuga uauuguuagc     300

cugguuacca ccguuuauga uaccaccgaa cugcgcaaua acaucaacaa aguuauugag     360

aaugccaacg acccgaaaug gucagaugau agccguaaag auguucugag caaaaucgag     420

gugaucaaaa acgauauuga uaacaacccg aaaacccaga gcgauaucga caacaaaauu     480

guggaaguga acgagcugga aaaacugcug guucugccgg aauuuagcac cauuccgggu     540

ucagcagcca cacugaauac cagcauuacc aaaaacauuc agaauggcaa cgccuacauu     600

gaucuguacg auguaaaaaa uggucugauc gauccgcaga accugaucgu gcugaauccg 660

agcagcuauu cagccaauua uuauauuaaa caaggcgcaa aauacuauag caacccgagc 720

gaaauuacca ccaccgguag cgccaccauu acguuuaaua uccuggacga aaccgguaac 780

ccgcauaaaa aagcagaugg ucaaauugau aucgugagcg uuaaccugac gauuuaugau 840

agcacagccc ugcguaaucg uauugaugaa gugauuaaua acgcgaauga uccuaaaugg 900

uccgauggua gucgugauga aguacugacc ggucuggaaa aaaucaaaaa agacaucgac 960

aauaauccga aaacgcagau ugacauugac aauaaaauca acgaggugaa cgagaucgag 1020

aaacugcugg uaguuagccu guaauaa 1047

<210> 40
<211> 1047
<212> DNA
<213> Streptococcus sp.

<400> 40
atggccgaag tgatctccgg ctctgctgct accctgaact ctgccctggt caagaatgtg 60

tctggcggca aggcctacat cgacatctac gacgtgaaga acggcaagat cgaccctctg 120

aacctgatcg tgctgacccc ttccaactac tccgccaact actacatcaa gcaaggcggc 180

agaatcttca cctccgtgaa ccagctgcag acccctggca ccgctaccat cacctacaac 240

atcctggacg agaatggcaa cccctacacc aagtccgacg gccagatcga tatcgtgtcc 300

ctggtcacca ccgtgtacga caccaccgag ctgcggaaca acatcaacaa agtgatcgag 360

aacgccaacg atcccaagtg gagcgacgac tctagaaagg acgtgctgtc caagatcgaa 420

gtcatcaaga acgacatcga caacaacccc aagacacagt ccgacattga taacaagatc 480

gtggaagtga acgagctgga aaagctgctg gtgctgcccg agttcagcac aatccctgga 540

tctgccgcca cactgaacac ctccatcacc aagaatatcc agaacgggaa cgcttacatc 600

gacctgtatg atgtcaagaa cgggctgatc gatccccaga atctgattgt gctcaacccc 660

tcctcctact ctgctaatta ctatattaag cagggcgcca agtactactc taacccctcc 720

gagatcacca ccaccggctc cgccaccatc acattcaata ttctggatga gacaggcaac 780

cctcacaaga aggctgacgg acagattgac attgtgtctg tgaacctgac catctacgat 840

tccaccgctc tgcggaaccg gatcgatgaa gtgatcaaca acgctaatga ccctaagtgg 900

agtgacggct ccagagatga ggtgctgacc ggcctggaaa agatcaagaa ggatatcgat 960

aacaatccta aaactcagat cgatattgac aacaagatca acgaagtcaa cgagatcgag 1020

aagctcctgg tcgtgtctct gtgatga 1047

<210> 41
<211> 1047

<212>  RNA
<213>  Streptococcus sp.

<400>  41
auggccgaag ugaucuccgg cucugcugcu acccugaacu cugcccuggu caagaaugug      60

ucuggcggca aggccuacau cgacaucuac gacgugaaga acggcaagau cgacccucug     120

aaccugaucg ugcugacccc uuccaacuac uccgccaacu acuacaucaa gcaaggcggc     180

agaaucuuca ccuccgugaa ccagcugcag accccuggca ccgcuaccau caccuacaac     240

auccuggacg agaauggcaa ccccuacacc aaguccgacg gccagaucga uaucgugucc     300

cuggucacca ccguguacga caccaccgag cugcggaaca acaucaacaa agugaucgag     360

aacgccaacg aucccaagug gagcgacgac ucuagaaagg acgugcuguc caagaucgaa     420

gucaucaaga acgacaucga caacaacccc aagacacagu ccgacauuga uaacaagauc     480

guggaaguga acgagcugga aaagcugcug gugcugcccg aguucagcac aaucccugga     540

ucugccgcca cacugaacac cuccaucacc aagaauaucc agaacgggaa cgcuuacauc     600

gaccuguaug augucaagaa cgggcugauc gauccccaga aucugauugu gcucaacccc     660

uccuccuacu cugcuaauua cuauauuaag cagggcgcca aguacuacuc uaaccccucc     720

gagaucacca ccaccggcuc cgccaccauc acauucaaua uucuggauga gacaggcaac     780

ccucacaaga aggcugacgg acagauugac auugucugug ugaaccugac caucuacgau     840

uccaccgcuc ugcggaaccg gaucgaugaa gugaucaaca acgcuaauga cccuaagugg     900

agugacggcu ccagagauga ggugcugacc ggccuggaaa agaucaagaa ggauaucgau     960

aacaauccua aaacucagau cgauauugac aacaagauca acgaagucaa cgagaucgag    1020

aagcuccugg ucgugucucu gugauga                                        1047


<210>  42
<211>  347
<212>  PRT
<213>  Streptococcus sp.

<400>  42

Met Ala Glu Val Ile Ser Gly Ser Ala Ala Thr Leu Asn Ser Ala Leu
1               5                   10                  15


Val Lys Asn Val Ser Gly Gly Lys Ala Tyr Ile Asp Ile Tyr Asp Val
            20                  25                  30


Lys Asn Gly Lys Ile Asp Pro Leu Asn Leu Ile Val Leu Thr Pro Ser
        35                  40                  45


Asn Tyr Ser Ala Asn Tyr Tyr Ile Lys Gln Gly Gly Arg Ile Phe Thr
    50                  55                  60

Ser Val Asn Gln Leu Gln Thr Pro Gly Thr Ala Thr Ile Thr Tyr Asn
65                  70                  75                  80

Ile Leu Asp Glu Asn Gly Asn Pro Tyr Thr Lys Ser Asp Gly Gln Ile
                85                  90                  95

Asp Ile Val Ser Leu Val Thr Thr Val Tyr Asp Thr Thr Glu Leu Arg
            100                 105                 110

Asn Asn Ile Asn Lys Val Ile Glu Asn Ala Asn Asp Pro Lys Trp Ser
        115                 120                 125

Asp Asp Ser Arg Lys Asp Val Leu Ser Lys Ile Glu Val Ile Lys Asn
    130                 135                 140

Asp Ile Asp Asn Asn Pro Lys Thr Gln Ser Asp Ile Asp Asn Lys Ile
145                 150                 155                 160

Val Glu Val Asn Glu Leu Glu Lys Leu Leu Val Leu Pro Glu Phe Ser
                165                 170                 175

Thr Ile Pro Gly Ser Ala Ala Thr Leu Asn Thr Ser Ile Thr Lys Asn
            180                 185                 190

Ile Gln Asn Gly Asn Ala Tyr Ile Asp Leu Tyr Asp Val Lys Asn Gly
        195                 200                 205

Leu Ile Asp Pro Gln Asn Leu Ile Val Leu Asn Pro Ser Ser Tyr Ser
    210                 215                 220

Ala Asn Tyr Tyr Ile Lys Gln Gly Ala Lys Tyr Tyr Ser Asn Pro Ser
225                 230                 235                 240

Glu Ile Thr Thr Thr Gly Ser Ala Thr Ile Thr Phe Asn Ile Leu Asp
                245                 250                 255

Glu Thr Gly Asn Pro His Lys Lys Ala Asp Gly Gln Ile Asp Ile Val
            260                 265                 270

Ser Val Asn Leu Thr Ile Tyr Asp Ser Thr Ala Leu Arg Asn Arg Ile
        275                 280                 285

Asp Glu Val Ile Asn Asn Ala Asn Asp Pro Lys Trp Ser Asp Gly Ser
    290                 295                 300

Arg Asp Glu Val Leu Thr Gly Leu Glu Lys Ile Lys Lys Asp Ile Asp
305                 310                 315                 320

Asn Asn Pro Lys Thr Gln Ile Asp Ile Asp Asn Lys Ile Asn Glu Val
                325                 330                 335

Asn Glu Ile Glu Lys Leu Leu Val Val Ser Leu
            340                 345